# EUROPEAN PATENT SPECIFICATION

(11) **EP 4 405 026 B1**
(45) Date of publication and mention of the grant of the patent: **02.12.2026**
(21) Application number: 22793040.1
(22) Date of filing: 27.09.2022
(51) Int. Cl.: A61M 25/06

(54) **COUPLERS AND ASSEMBLIES THEREOF FOR RAPIDLY INSERTABLE CENTRAL CATHETERS**
KOPPLER UND ANORDNUNGEN DAVON FÜR SCHNELL EINSETZBARE ZENTRALKATHETER
COUPLEURS ET ENSEMBLES DE CEUX-CI POUR CATHÉTERS CENTRAUX À INSERTION RAPIDE

(30) Priority: 27.09.2021 US 202163249009 P; 22.10.2021 US 202163271043 P; 06.04.2022 US 202263328179 P
(43) Date of publication of application: 31.07.2024
(73) Proprietor: Bard Access Systems, Inc., Salt Lake City, UT 84116 (US)
(72) Inventor: WEST, Sharon, Elkins Park, PA 19027 (US); BECHSTEIN, Justin, Philadelphia, PA 19147 (US); BIVIANO, Carter, Philadelphia, PA 19121 (US); MURRAY, Christopher, J., Philadelphia, PA 19147 (US); HOWELL, Glade, H., Draper, UT 84020 (US); BLANCHARD, Daniel, B., Bountiful, UT 84010 (US); SPATARO, Joe, Cottonwood Heights, UT 84121 (US); THORNLEY, Kyle, G., Farmington, UT 84025 (US)
(74) Representative: Hoffmann Eitle
(86) International application number: PCT/US2022/044923
(87) International publication number: WO 2023/049522

(56) References cited:
- EP-A1- 3 473 291
- US-A- 5 380 290
- US-A1- 2009 187 147
- US-A1- 2021 085 927

## Description

### PRIORITY

This application claims the benefit of priority to U.S. Provisional Application No. 63/249,009, filed September 27, 2021; U.S. Provisional Application No. 63/271,043, filed October 22, 2021; and U.S. Provisional Application No. 63/328,179, filed April 6, 2022.

### BACKGROUND

Central venous catheter ("CVCs") are commonly introduced into patients and advanced through their vasculatures by way of the Seldinger technique. The Seldinger technique utilizes a number of steps and medical devices (e.g., a needle, a scalpel, a guidewire, an introducer sheath, a dilator, a CVC, etc.). While the Seldinger technique is effective, the number of steps are time consuming, handling the number of medical devices is awkward, and both of the foregoing can lead to patient trauma. In addition, there is a relatively high potential for touch contamination due to the number of medical devices that need to be interchanged during the Seldinger technique. As such, there is a need to reduce the number of steps and medical devices involved in introducing a catheter such as a CVC into a patient and advancing the catheter through a vasculature thereof.

See, e.g., US2021085927 A1.

Disclosed herein are couplers and assemblies thereof for rapidly insertable central catheters ("RICCs") that address the foregoing.

### SUMMARY OF THE INVENTION

In a first aspect of the present invention, there is provided an introducer needle assembly according to claim 1.

In a second aspect of the present invention, there is provided a RICC insertion assembly according to claim 11.

The dependent claims define preferred embodiments.

### SUMMARY OF THE DISCLOSURE

Disclosed herein is a RICC insertion assembly including, in some embodiments, a RICC, an introducer needle, an access guidewire, and a coupler coupling the RICC, the introducer needle, and the access guidewire together. The introducer needle includes a needle shaft, a sheath, and a needle hub over proximal portions of the needle shaft and sheath. The needle shaft includes a longitudinal needle slot extending from a proximal portion of the needle shaft through a distal needle tip. The sheath is over the needle shaft sealing the needle slot thereunder except for the needle slot under a sheath opening in a proximal portion of the sheath. The needle hub includes a distally extending needle-hub extension arm. In addition, the needle hub includes a needle-hub portion of a RICC clip configured to hold a portion of the RICC therein in at least a ready-to-deploy state of the RICC insertion assembly. The access guidewire includes a proximal portion including a proximal end and a distal portion including a distal end. The distal end of the access guidewire is disposed in the introducer needle just proximal of the needle tip in at least the ready-to-deploy state of the RICC insertion assembly. The coupler includes a coupler housing and a coupler portion of the RICC clip connected to the coupler housing. The proximal and distal ends of the access guidewire enforce a loop in the access guidewire over which the RICC is disposed in at least the ready-to-deploy state of the RICC insertion assembly.

In some embodiments, the coupler housing includes a proximally extending coupler-housing support. At least the needle-hub extension arm is slidably disposed over the coupler-housing support in at least the ready-to-deploy state of the RICC insertion assembly.

In some embodiments, the coupler portion of the RICC clip is a first 'C'-shaped portion of the RICC clip suspended from a proximal portion of the coupler-housing support. The needle-hub portion of the RICC clip is a second 'C'-shaped portion of the RICC clip suspended from a proximal portion of the needle hub. The first and second 'C'-shaped portions of the RICC clip open toward each other such that the RICC clip is completed in at least the ready-to-deploy state of the RICC insertion assembly.

In some embodiments, the RICC clip is configured to hold one or more extension legs of the RICC therein such that the RICC is suspended from the RICC insertion assembly in at least the ready-to-deploy state of the RICC insertion assembly.

In some embodiments, the RICC clip is configured to release the one-or-more extension legs of the RICC therefrom when the introducer needle is withdrawn from the coupler.

In some embodiments, the coupler housing includes a needle-hub receptacle in a proximal portion of the coupler housing. A distal portion of the needle hub includes the needle-hub extension arm disposed in the needle-hub receptacle in at least the ready-to-deploy state of the RICC insertion assembly.

In some embodiments, the coupler further includes a valve module disposed in a valve-module compartment of the coupler housing distal of the needle-hub receptacle. The valve module seals around the proximal portion of the sheath and the distal portion of the access guidewire that extends through the sheath opening in at least the ready-to-deploy state of the RICC insertion assembly. The seals around the sheath and the access guidewire enable leak-free aspiration through the introducer needle.

In some embodiments, the valve module includes an integrated blade disposed in the needle slot under a distal end of the sheath opening. The blade includes a distal-facing blade edge configured to cut the sheath off the needle shaft when the introducer needle is withdrawn from the coupler. With the cutting of the sheath off the needle shaft, the access guidewire is allowed to escape from the needle shaft by way of the needle slot thereof.

In some embodiments, the coupler includes a pair of lock buttons extending through opposite sides of the coupler housing. The pair of lock buttons are respectively mounted on a pair of axles captively held in the coupler housing such that each lock button of the pair of lock buttons pivots on a corresponding axle.

In some embodiments, each lock button of the pair of lock buttons has a protrusion extending from a crossarm connecting the protrusion to an external, pressable portion of the lock button. In addition, each lock button of the pair of lock buttons has a gap opposite the crossarm between the protrusion and the external, pressable portion of the lock button.

In some embodiments, the pair of lock buttons are biased away from a centerline of the coupler such that the protrusion of each lock button of the pair of lock buttons is disposed in a notch of a pair of notches in a wall of the needle-hub extension arm on opposite sides of the needle-hub extension arm in a default state of the pair of lock buttons.

In some embodiments, the wall of the needle-hub extension arm on both sides of the needle-hub extension arm passes through the gap between the protrusion and the external, pressable portion of each lock button of the pair of lock buttons when the pair of lock buttons are pressed in toward the centerline of the coupler.

In some embodiments, the needle hub includes a proximally extending needle-hub cradle with grip pads on opposite sides of the needle-hub cradle to facilitate holding the RICC insertion assembly thereby when the introducer needle is withdrawn from the coupler.

In some embodiments, the RICC insertion assembly further includes a syringe fluidly coupled to the introducer needle in at least the ready-to-deploy state of the RICC insertion assembly. A barrel of the syringe is disposed in the needle-hub cradle with the grip pads flanking the barrel of the syringe.

In some embodiments, another RICC clip incorporated into a plunger-flange extension extends from a plunger flange of a plunger of the syringe. The other RICC clip is configured to hold a catheter hub of the RICC therein such that the RICC is suspended from the RICC insertion assembly in at least the ready-to-deploy state of the RICC insertion assembly.

In some embodiments, the needle hub includes an access-guidewire channel defined by a notch in a distally extending needle-hub extension tube over the proximal portions of the needle shaft and the sheath.

In some embodiments, the needle hub includes a transverse bar and a needle-hub pad between the bar and the access-guidewire channel. The bar and the needle-hub pad are configured for pressing and holding the access guidewire in place thereagainst when establishing a needle tract from an area of skin to a blood-vessel lumen of a patient.

In some embodiments, the coupler housing includes a longitudinal coupler-housing slot on a same side of the coupler housing as the coupler portion of the RICC clip. The coupler-housing slot opens in a direction opposite that of the needle slot of needle shaft. The coupler-housing slot is configured to allow the access guidewire to escape from the coupler housing when the introducer needle is withdrawn from the coupler.

In some embodiments, the coupler-housing slot has a narrower portion and a wider portion proximal of the narrower portion. The wider portion of the coupler-housing slot is configured for at least aligning a needle-hub alignment rail of the needle hub while assembling the RICC insertion assembly.

In some embodiments, the access guidewire includes an access-guidewire hub over the distal end of the access guidewire. The access-guidewire hub is configured to prevent over advancement of the access guidewire.

Also disclosed herein is an introducer-needle assembly including, in some embodiments, an introducer needle and a coupler coupled with the introducer needle. The introducer needle includes a needle shaft, a sheath, and a needle hub over proximal portions of the needle shaft and sheath. The needle shaft includes a longitudinal needle slot extending from a proximal portion of the needle shaft through a distal needle tip. The sheath is over the needle shaft sealing the needle slot thereunder except for the needle slot under a sheath opening in a proximal portion of the sheath. The needle hub includes a distally extending needle-hub extension arm. In addition, the needle hub includes a needle-hub portion of a RICC clip configured to hold a portion of the RICC therein. The coupler includes a coupler housing and a coupler portion of the RICC clip connected to the coupler housing.

In some embodiments, the coupler housing includes a proximally extending coupler-housing support. At least the needle-hub extension arm is slidably disposed over the coupler-housing support.

In some embodiments, the coupler portion of the RICC clip is a first 'C'-shaped portion of the RICC clip suspended from a proximal portion of the coupler-housing support. The needle-hub portion of the RICC clip is a second 'C'-shaped portion of the RICC clip suspended from a proximal portion of the needle hub. The first and second 'C'-shaped portions of the RICC clip open toward each other completing the RICC clip.

In some embodiments, the coupler housing includes a needle-hub receptacle in a proximal portion of the coupler housing. A distal portion of the needle hub includes the needle-hub extension arm disposed in the needle-hub receptacle.

In some embodiments, the coupler further includes a valve module disposed in a valve-module compartment of the coupler housing distal of the needle-hub receptacle. The valve module seals around the proximal portion of the sheath and, when present, a distal portion of an access guidewire that extends through the sheath opening. The seals around the sheath and the access guidewire enable leak-free aspiration through the introducer needle.

In some embodiments, the valve module includes an integrated blade disposed in the needle slot under a distal end of the sheath opening. The blade includes a distal-facing blade edge configured to cut the sheath off the needle shaft when the introducer needle is withdrawn from the coupler.

In some embodiments, the coupler includes a pair of lock buttons extending through opposite sides of the coupler housing. The pair of lock buttons are respectively mounted on a pair of axles captively held in the coupler housing such that each lock button of the pair of lock buttons pivots on a corresponding axle.

In some embodiments, each lock button of the pair of lock buttons has a protrusion extending from a crossarm connecting the protrusion to an external, pressable portion of the lock button. In addition, each lock button of the pair of lock buttons has a gap opposite the crossarm between the protrusion and the external, pressable portion of the lock button.

In some embodiments, the pair of lock buttons are biased away from a centerline of the coupler such that the protrusion of each lock button of the pair of lock buttons is disposed in a notch of a pair of notches in a wall of the needle-hub extension arm on opposite sides of the needle-hub extension arm in a default state of the pair of lock buttons.

In some embodiments, the wall of the needle-hub extension arm on both sides of the needle-hub extension arm passes through the gap between the protrusion and the external, pressable portion of each lock button of the pair of lock buttons when the pair of lock buttons are pressed in toward the centerline of the coupler.

Also disclosed herein is a method , not claimed, for inserting a RICC into a blood-vessel lumen of a patient. The method includes, in some embodiments, a RICC insertion assembly-obtaining step, a needle tract-establishing step, an access guidewire-advancing step, an introducer needle-withdrawing step, and a RICC-advancing step. The RICC insertion assembly-obtaining step includes obtaining a RICC insertion assembly including the RICC, an introducer needle including a sheath over a needle shaft, and an access guidewire coupled together by a coupler. In addition, a proximal portion of the access guidewire is disposed in a primary lumen of the RICC and a distal end of the access guidewire is disposed in the introducer needle by way of a valve module disposed in a coupler housing of the coupler. The proximal and distal ends of the access guidewire enforce a loop in the access guidewire over which the RICC is disposed in at least the ready-to-deploy state of the RICC insertion assembly. The needle tract-establishing step includes establishing a needle tract from an area of skin to the blood-vessel lumen with the introducer needle. The access guidewire-advancing step includes advancing the distal end of the access guidewire from its initial location in the needle shaft just proximal of a needle tip of the needle shaft into the blood-vessel lumen. The introducer needle-withdrawing step includes withdrawing the introducer needle by the needle hub from the coupler leaving the access guidewire in place in the blood-vessel lumen. A RICC clip formed between a needle-hub portion and a coupler portion of the RICC clip separates to allow a portion of the RICC held therein to escape with the withdrawing of the introducer needle from the coupler. The introducer needle includes a longitudinal needle slot extending from a proximal portion of the needle shaft through the needle tip allowing the access guidewire to escape therefrom with the withdrawing of the introducer needle from the coupler. The RICC-advancing step includes advancing a catheter tube of the RICC over the access guidewire for inserting the RICC into the blood-vessel lumen.

In some embodiments, the coupler portion of the RICC clip is a first 'C'-shaped portion of the RICC clip suspended from a proximal portion of a proximally extending coupler-housing support. The needle-hub portion of the RICC clip is a second 'C'-shaped portion of the RICC clip suspended from a proximal portion of a proximally extending needle-hub cradle. The first and second 'C'-shaped portions of the RICC clip open toward each other such that the RICC clip is completed in at least the ready-to-deploy state of the RICC insertion assembly.

In some embodiments, the method further includes a needle hub-unlocking step. The needle hub-unlocking step includes unlocking the needle hub before the withdrawing of the introducer needle from the coupler in the introducer needle-withdrawing step. The unlocking of the needle hub includes pressing a pair of lock buttons extending through opposite sides of the coupler housing into the coupler housing, thereby allowing a wall of a distally extending needle-hub extension arm to pass through a gap between a protrusion and an external, pressable portion of a lock button for each lock button of the pair of lock buttons.

In some embodiments, the needle tract-establishing step includes pressing and holding the access guidewire in place against a transverse bar or a needle-hub pad of the needle hub. The needle-hub pad is between the bar and an access-guidewire channel defined by a notch in a distally extending needle-hub extension tube.

These and other features of the concepts provided herein will become more apparent to those of skill in the art in view of the accompanying drawings and following description, which describe particular embodiments of such concepts in greater detail.

### DRAWINGS

FIG. 1 illustrates a side view of a RICC insertion assembly in accordance with some embodiments.
FIG. 2 illustrates a perspective view of an alternative RICC insertion assembly in accordance with some embodiments.
FIG. 3 illustrates a perspective view of a top of an introducer-needle assembly including a syringe in accordance with some embodiments.
FIG. 4 illustrates a perspective view of a bottom of the introducer-needle assembly and the syringe in accordance with some embodiments.
FIG. 5 illustrates a side view of the introducer-needle assembly in accordance with some embodiments.
FIG. 6 illustrates a top view of the introducer-needle assembly in accordance with some embodiments.
FIG. 7 illustrates a bottom view of the introducer-needle assembly in accordance with some embodiments.
FIG. 8 illustrates a side view of the introducer-needle assembly with an introducer needle being withdrawn from a coupler in accordance with some embodiments.
FIG. 9 illustrates a top view of the introducer-needle assembly with the introducer needle being withdrawn from the coupler in accordance with some embodiments.
FIG. 10 illustrates a bottom view of the introducer-needle assembly with the introducer needle being withdrawn from the coupler in accordance with some embodiments.
FIG. 11 illustrates a perspective view of the introducer-needle assembly in accordance with some embodiments, wherein half of a coupler housing and half of a needle hub are removed to assist in visualizing certain features of the introducer-needle assembly.
FIG. 12 illustrates a perspective view of the introducer-needle assembly in accordance with some embodiments, wherein the coupler housing is removed to assist in visualizing certain features of the introducer-needle assembly.
FIG. 13 illustrates a side view of a valve module of the coupler over both a sheath and needle shaft of the introducer needle in accordance with some embodiments.
FIG. 14 illustrates a longitudinal cross section of the valve module of the coupler over both the sheath and needle shaft of the introducer needle in accordance with some embodiments.
FIG. 15 illustrates a side view of the introducer needle in accordance with some embodiments.
FIG. 16 illustrates a top view of the introducer needle in accordance with some embodiments.
FIG. 17 illustrates a top view of the sheath over the needle shaft of the introducer needle in accordance with some embodiments.
FIG. 18 illustrates a top view of the sheath in accordance with some embodiments.
FIG. 19 illustrates a top view of the needle shaft in accordance with some embodiments.
FIG. 20 illustrates a RICC of the RICC insertion assembly in accordance with some embodiments.
FIG. 21 illustrates a detailed view of a distal portion of a catheter tube of the RICC in accordance with some embodiments.
FIG. 22 illustrates a transverse cross section of the distal portion of the catheter tube in accordance with some embodiments.
FIG. 23 illustrates another transverse cross section of the distal portion of the catheter tube in accordance with some embodiments.
FIG. 24 illustrates a longitudinal cross section of the distal portion of the catheter tube in accordance with some embodiments.

### DESCRIPTION

Before some particular embodiments are disclosed in greater detail, it should be understood that the particular embodiments disclosed herein do not limit the scope of the concepts provided herein. It should also be understood that a particular embodiment disclosed herein can have features that can be readily separated from the particular embodiment and optionally combined with or substituted for features of any of a number of other embodiments disclosed herein.

Regarding terms used herein, it should also be understood the terms are for the purpose of describing some particular embodiments, and the terms do not limit the scope of the concepts provided herein. Ordinal numbers (e.g., first, second, third, etc.) are generally used to distinguish or identify different features or steps in a group of features or steps, and do not supply a serial or numerical limitation. For example, "first," "second," and "third" features or steps need not necessarily appear in that order, and the particular embodiments including such features or steps need not necessarily be limited to the three features or steps. In addition, any of the foregoing features or steps can, in turn, further include one or more features or steps unless indicated otherwise. Labels such as "left," "right," "top," "bottom," "front," "back," and the like are used for convenience and are not intended to imply, for example, any particular fixed location, orientation, or direction. Instead, such labels are used to reflect, for example, relative location, orientation, or directions. Singular forms of "a," "an," and "the" include plural references unless the context clearly dictates otherwise.

With respect to "proximal," a "proximal portion" or a "proximal-end portion" of, for example, a catheter includes a portion of the catheter intended to be near a clinician when the catheter is used on a patient. Likewise, a "proximal length" of, for example, the catheter includes a length of the catheter intended to be near the clinician when the catheter is used on the patient. A "proximal end" of, for example, the catheter includes an end of the catheter intended to be near the clinician when the catheter is used on the patient. The proximal portion, the proximal-end portion, or the proximal length of the catheter can include the proximal end of the catheter; however, the proximal portion, the proximal-end portion, or the proximal length of the catheter need not include the proximal end of the catheter. That is, unless context suggests otherwise, the proximal portion, the proximal-end portion, or the proximal length of the catheter is not a terminal portion or terminal length of the catheter.

With respect to "distal," a "distal portion" or a "distal-end portion" of, for example, a catheter includes a portion of the catheter intended to be near or in a patient when the catheter is used on the patient. Likewise, a "distal length" of, for example, the catheter includes a length of the catheter intended to be near or in the patient when the catheter is used on the patient. A "distal end" of, for example, the catheter includes an end of the catheter intended to be near or in the patient when the catheter is used on the patient. The distal portion, the distal-end portion, or the distal length of the catheter can include the distal end of the catheter; however, the distal portion, the distal-end portion, or the distal length of the catheter need not include the distal end of the catheter. That is, unless context suggests otherwise, the distal portion, the distal-end portion, or the distal length of the catheter is not a terminal portion or terminal length of the catheter.

Unless defined otherwise, all technical and scientific terms used herein have the same meaning as commonly understood by those of ordinary skill in the art.

As set forth above with respect to the Seldinger technique, the number of steps are time consuming, handling the number of medical devices is awkward, and both of the foregoing can lead to patient trauma. In addition, there is a relatively high potential for touch contamination due to the number of medical devices that need to be interchanged during the Seldinger technique. As such, there is a need to reduce the number of steps and medical devices involved in introducing a catheter such as a CVC into a patient and advancing the catheter through a vasculature thereof.

Disclosed herein are couplers and assemblies thereof for RICCs. For example, an introducer-needle assembly for a RICC can include an introducer needle and a coupler coupled therewith. The introducer needle can include a needle having a longitudinal needle slot extending from a proximal portion of the needle shaft through a distal needle tip. A sheath can be over the needle shaft sealing the needle slot thereunder except for a portion of the needle slot under a sheath opening in a proximal portion of the sheath. A needle hub can include a distally extending needle-hub extension arm over the proximal portions of the needle shaft and sheath. The needle hub can also include a needle-hub portion of a RICC clip configured to hold a portion of the RICC therein. The coupler can include a complementary coupler portion of the RICC clip connected to a coupler housing.

The foregoing features as well as other features of the couplers and assemblies thereof for the RICCs will become more apparent to those of skill in the art in view of the accompanying drawings and following description, which describe particular embodiments of the foregoing in the context of RICC insertion assemblies. Notably, the RICC insertion assemblies include but one type of catheter that can be incorporated into such catheter insertion assemblies. It should be understood peripherally inserted central catheters ("PICCs"), dialysis catheters, or the like can likewise be incorporated into catheter insertion assemblies like the RICC insertion assemblies set forth below.

### RICC insertion assemblies

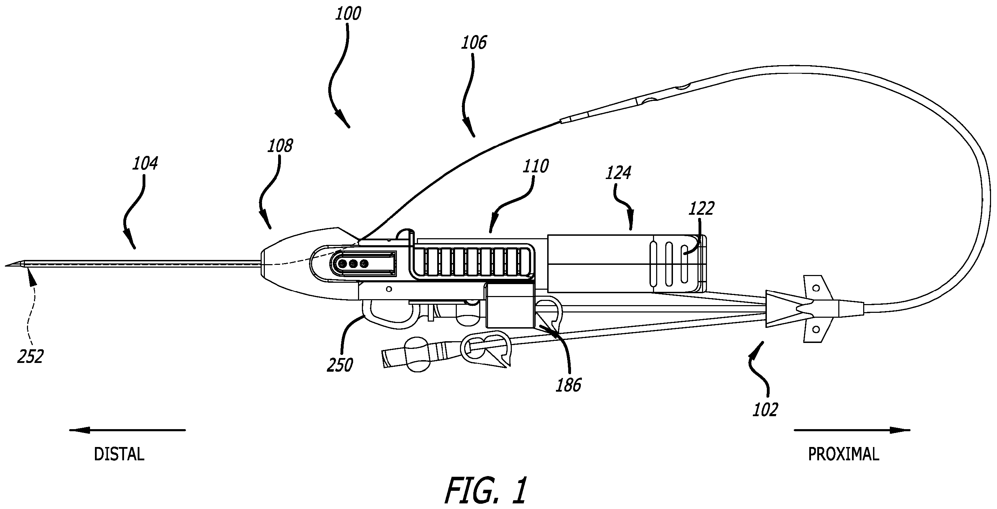

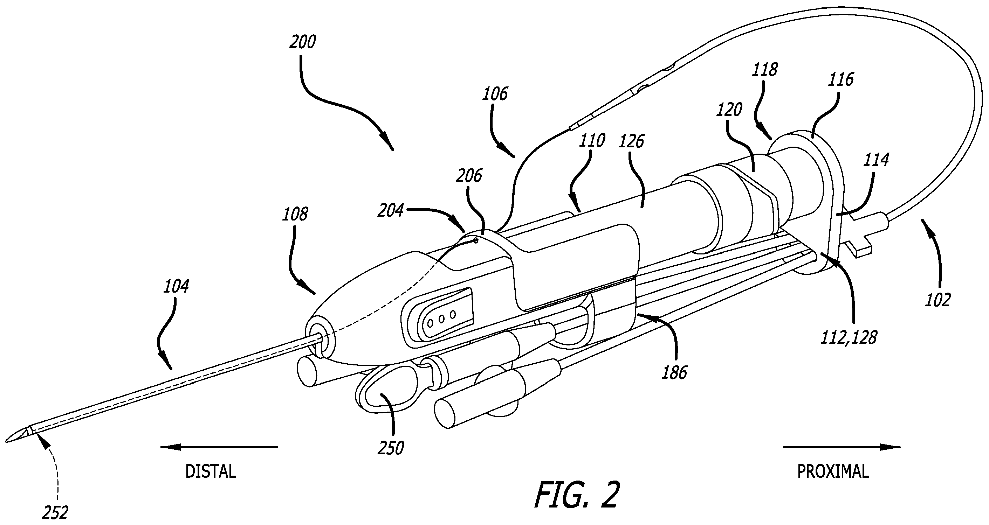

FIG. 1 illustrates a RICC insertion assembly 100 in accordance with some embodiments. FIG. 2 illustrates an alternative RICC insertion assembly 200 in accordance with some embodiments. For brevity, genus-type features (e.g., the needle hub 160 of the introducer needle 104) among the RICC insertion assemblies 100 and 200 or subassemblies thereof are referred to herein by a single reference number even though each RICC insertion assembly of the RICC insertion assemblies 100 and 200 or a subassembly thereof might include species-type features (e.g., the bar 198 and the needle-hub pad 202 of the needle hub 160 shown in FIG. 1 for the RICC insertion assembly 100 versus the pinch holder 204 of the needle hub 160 shown in FIG. 2 for the RICC insertion assembly 200) that differentiate a particular genus-type feature from that of another RICC insertion assembly or subassembly. In addition, the RICC insertion assembly 100 is primarily referred to herein for convenience; however, it should be understood description for the RICC insertion assembly 100 extends to the RICC insertion assembly 200 unless context suggest otherwise.

As shown, the RICC insertion assembly 100 includes a RICC 102, an introducer needle 104, an access guidewire 106, and a coupler 108 coupling the RICC 102, the introducer needle 104, and the access guidewire 106 together in at least a ready-to-deploy state of the RICC insertion assembly 100. In the ready-to-deploy state of the RICC insertion assembly 100, as set forth below, the proximal end of the access guidewire 106 is disposed in the access-guidewire hub 250 coupled to a Luer connector of the RICC 102 and the distal end of the access guidewire 106 is disposed in the needle lumen 174 of the introducer needle 104. This enforces a loop in the access guidewire 106. The RICC 102 is disposed over the loop in the ready-to-deploy state of the RICC insertion assembly 100 keeping the RICC insertion assembly 100 in a relatively compact form.

The RICC insertion assembly 100 can further include a syringe 110 fluidly coupled to the introducer needle 104 in at least the ready-to-deploy state of the RICC insertion assembly 100. As set forth below, the sheath 164 seals the needle slot 168 of the needle shaft 162. In particular, the sheath 164 seals the needle slot 168 outside of the valve module 218. The valve module 218, in turn, seals over the sheath opening 178 of the sheath 164 that opens to the needle slot 168. The valve module 218 also seals around the access guidewire 106. Such seals enable the syringe 110 to aspirate blood in accordance with the blood-aspirating step of the method set forth below.

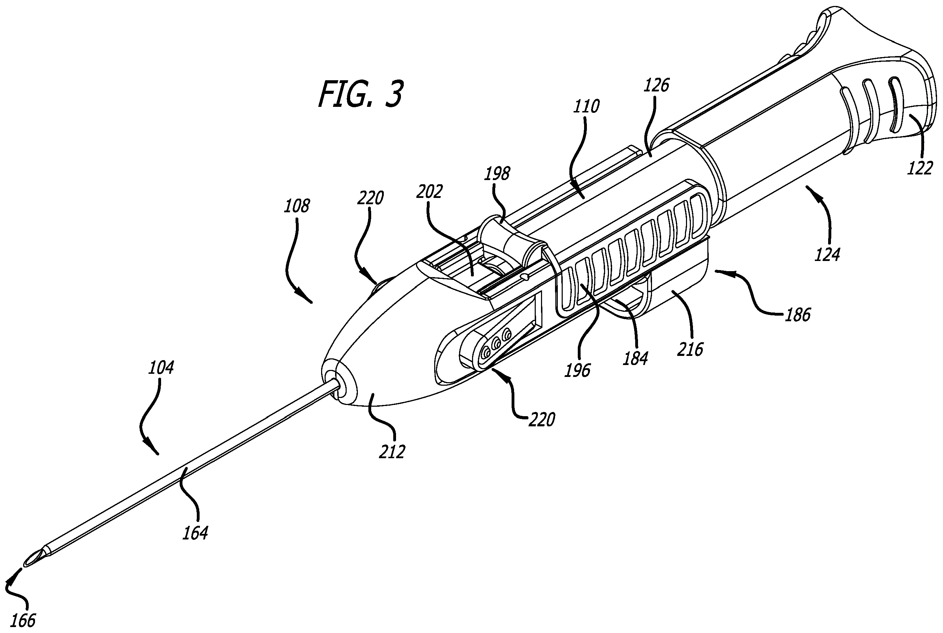

Notably, a RICC clip 112 in addition to the RICC clip 186 set forth below, or as an alternative thereto, can be incorporated into the syringe 110 by way of a suitably configured plunger-flange extension 114 for holding the catheter hub 132 of the RICC 102 therein. As shown in FIG. 2, the plunger-flange extension 114 can extend from a plunger flange 116 of a plunger stopper 118 inserted into a proximal portion of a tubular plunger 120. In an alternative to the foregoing, the plunger-flange extension 114 can instead extend from a plunger flange that is integral with a plunger like that commonly seen in disposable syringes having plungers with orthogonal longitudinal support members. In another alternative to the foregoing, the plunger-flange extension 114 can instead extend from an optionally textured plunger flange 122 that is incorporated into a plunger-coupled barrel cap 124 slidably disposed over a barrel 126 of the syringe 110. (*See* FIG. 3 for the plunger flange 122 of the barrel cap 124.) Regardless, the plunger-flange extension 114 includes a plunger-flange extension slot 128 therein, thereby forming the RICC clip 112, which is configured to hold the catheter hub 132 of the RICC 102 therein such that the RICC 102 is suspended from the RICC insertion assembly 100 or 200 in at least the ready-to-deploy state of the RICC insertion assembly 100 or 200.

Lastly, any component of the RICC insertion assembly 100 selected from at least the RICC 102, the introducer needle 104, the access guidewire 106, the coupler 108, and the syringe 110, or any portion of the component selected from the foregoing components, can include an antimicrobial thereon or therein. In an example, the catheter tube 130 of the RICC 102 can include an antimicrobial coating on an abluminal surface of the catheter tube 130, a luminal surface of the catheter tube 130, or both. In another example, a pre-extrusion material of the catheter tube 130 can include the antimicrobial admixed therein such that the antimicrobial is incorporated into the catheter tube 130 when extruded, the antimicrobial protecting both the abluminal surface of the catheter tube 130 and the luminal surface of the catheter tube 130 from microbial contamination.

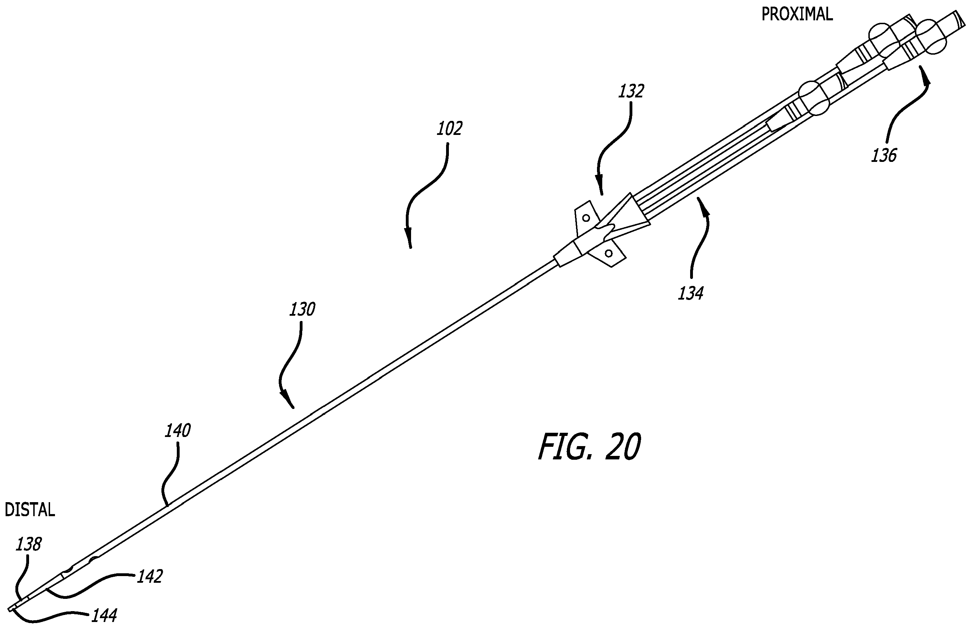

FIG. 20 illustrates the RICC 102 of the RICC insertion assembly 100 in accordance with some embodiments.

As shown, the RICC 102 includes a catheter tube 130, a catheter hub 132, one or more extension legs 134, and one or more extension-leg connectors 136.

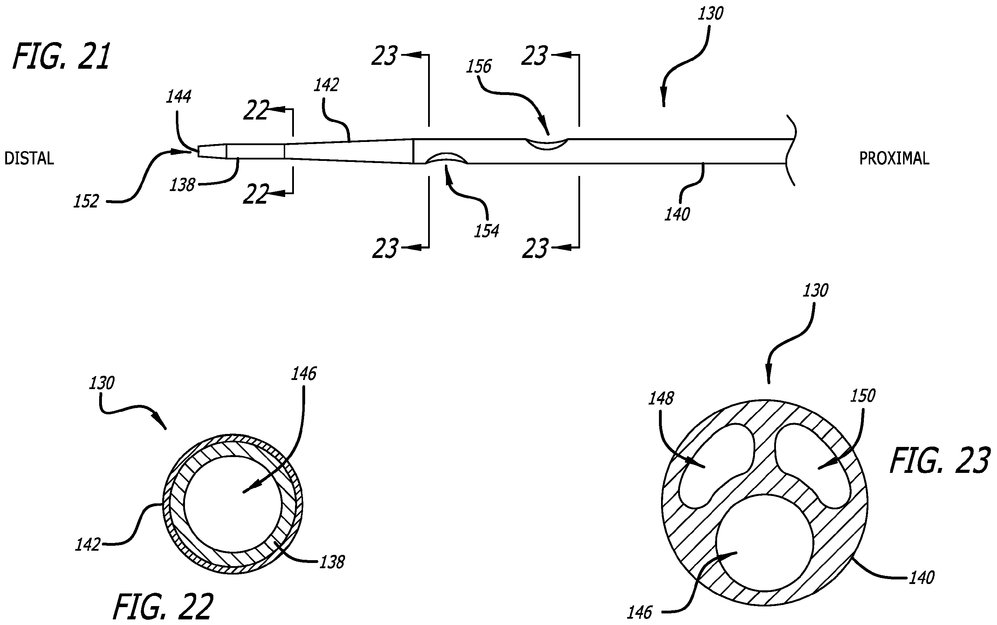

FIGS. 21-24 illustrate various views of the catheter tube 130 of the RICC 102 in accordance with some embodiments.

The catheter tube 130 includes a first section 138 in a distal portion of the catheter tube 130, a second section 140 in the distal portion of the catheter tube 130 proximal of the first section 138, and a tapered junction 142 between the first and second sections 138 and 140 of the catheter tube 130.

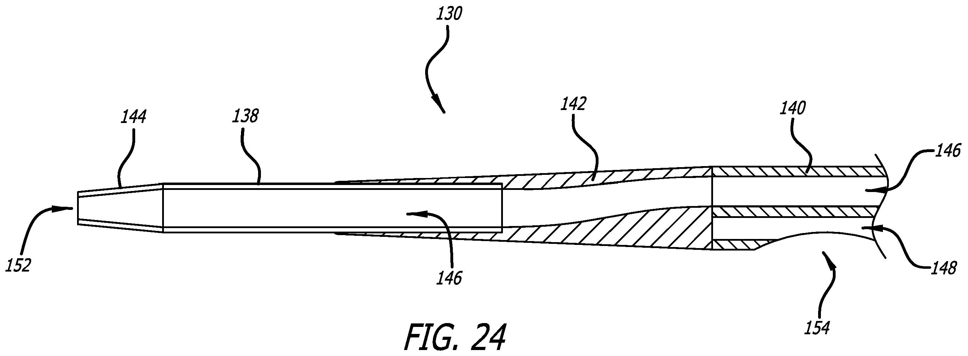

The first section 138 of the catheter tube 130 includes a catheter tip 144 having a relatively short taper from an outer diameter of a distal portion of the first section 138 distal of the junction 142 to an outer diameter of a distal end of the first section 138. The taper of the catheter tip 144 is configured for immediate dilation of tissue about a needle tract established with the introducer needle 104 up to the outer diameter of the distal portion of the first section 138 of the catheter tube 130. As best shown in FIG. 24, the first section 138 of the catheter tube 130 also includes a proximal portion disposed in a bore of a distal portion of the junction 142 and fixedly coupled thereto such as by a solvent bond, an adhesive bond, or a heat weld.

The second section 140 of the catheter tube 130 includes a consistent outer diameter over its length from a distal end of the second section 140 to a proximal end of the second section 140. The consistent diameter of the second section 140 of the catheter tube 130 is configured for smooth insertion into the needle tract and targeted vasculature subsequent to any dilation by the first section 138 of the catheter tube 130 and the junction 142. The distal end of the second section 140 of the catheter tube 130 has a flat face flush with the flat-faced proximal end of the junction 142 and fixedly coupled thereto such as by a solvent bond, an adhesive bond, or a heat weld.

The junction 142 includes a taper over its length from a proximal end of the junction 142 to a distal end of the junction 142. The taper of the junction 142 is configured for immediate dilation of the tissue about the needle tract from the outer diameter of the proximal portion of the first section 138 of the catheter tube 130 to the outer diameter of the second section 140 of the catheter tube 130. An abluminal surface of the junction 142 smoothly transitions from an abluminal surface of the first section 138 of the catheter tube 130 to an abluminal surface of the second section 140 of the catheter tube 130 without edges that catch on skin when the catheter tube 130 is inserted into the needle tract. In addition to the edges being minimal to negligible, the edges can include solvent-interdiffused polymeric material of the polymeric materials from which the catheter tube 130 is formed, which smoothens the transitions from the first section 138 of the catheter tube 130 to the junction 142 and from the junction 142 to the second section 140 of the catheter tube 130. Notably, the junction 142 has a length approximately commensurate with a length of an exposed portion of the first section 138 of the catheter tube 130 or between lengths of exposed portions of the first and second sections 138 and 140 of the catheter tube 130. As such, the length of the exposed portion of the first section 138 of the catheter tube 130 is less than the length of the junction 142 up to approximately commensurate with the length of the junction 142.

The first section 138 of the catheter tube 130 is formed of a first polymeric material (e.g., a polytetrafluoroethylene, a polypropylene, or a polyurethane) having a first durometer. The second section 140 of the catheter tube 130 is formed of a second polymeric material (e.g., a polyvinyl chloride, a polyethylene, another polyurethane, or a silicone) having a second durometer less than the first durometer. For example, the first section 138 of the catheter tube 130 can be formed of a first polyurethane having the first durometer while the second section 140 of the catheter tube 130 can be formed of a second, different polyurethane (e.g., a same or different diisocyanate or triisocyanate reacted with a different diol or triol, a different diisocyanate or triisocyanate reacted with a same or different diol or triol, a same diisocyanate or triisocyanate reacted with a same diol or triol under different conditions or with different additives, etc.) having the second durometer less than the first durometer. Indeed, polyurethanes are advantageous for the catheter tube 130 in that polyurethanes can be relatively rigid at room-temperature but become more flexible *in vivo* at body temperature, which reduces irritation to vessel walls as well as phlebitis. Polyurethanes are also advantageous in that they can be less thrombogenic than some other polymers. The junction 142 is formed of the second polymeric material or a third polymeric material (e.g., yet another polyurethane) having a third durometer less than the first durometer and greater than, approximately equal to, or less than the second durometer.

It should be understood the first durometer of the first polymeric material, the second durometer of the second polymeric material, and the third durometer of the third polymeric material can be on different scales (e.g., Type A or Type D). With this understanding, the second durometer of the second polymeric material or the third durometer of the third polymeric material might not be numerically less than the first durometer of the first polymeric material when the second durometer or the third durometer is less than the first durometer. Indeed, the hardness of the second polymeric material or the third polymeric material can still be less than the hardness of the first polymeric material as the different scales-each of which ranges from 0 to 100-are designed for characterizing different materials in groups of the materials having a like hardness.

In accordance with the first section 138 of the catheter tube 130, the second section 140 of the catheter tube 130, and the junction 142 between the first and second sections 138 and 140 of the catheter tube 130 set forth above, the catheter tube 130 possesses a column strength sufficient to prevent buckling of the catheter tube 130 when inserted into a needle tract established with the introducer needle 104. The column strength of the catheter tube 130 is also sufficient to prevent buckling of the catheter tube 130 when advanced through a vasculature of a patient without dilation of tissue about the needle tract or any blood vessels of the vasculature beforehand with a separate dilator.

The catheter tube 130 includes one or more catheter-tube lumens extending through the catheter tube 130; however, only one catheter-tube lumen typically extends from a proximal end of the catheter tube 130 to a distal end of the catheter tube 130 in a multiluminal RICC (e.g., a diluminal RICC, a triluminal RICC, a tetraluminal RICC, a pentaluminal RICC, a hexaluminal RICC, etc.). (*See* FIGS. 21-24.) Indeed, the first section 138 of the catheter tube 130 typically includes a single lumen therethrough as best shown in FIG. 24.

The catheter hub 132 is coupled to a proximal portion of the catheter tube 130. The catheter hub 132 includes one or more catheter-hub lumens corresponding in number to the one-or-more catheter-tube lumens. The one-or-more catheter-hub lumens extends through an entirety of the catheter hub 132 from a proximal end of the catheter hub 132 to a distal end of the catheter hub 132.

Each extension leg of the one-or-more extension legs 134 is coupled to the catheter hub 132 by a distal portion thereof. The one-or-more extension legs 134 respectively include one or more extension-leg lumens, which, in turn, correspond in number to the one-or-more catheter-hub lumens. Each extension-leg lumen of the one-or-more extension-leg lumens extends through an entirety of the extension leg from a proximal end of the extension leg to a distal end of the extension leg.

Each extension-leg connector of the one-or-more extension-leg connectors 136 is over a proximal portion of an extension leg of the one-or-more extension legs 134. For example, each extension-leg connector of the one-or-more extension-leg connectors 136 can be a Luer connector over a proximal portion of an extension leg of the one-or-more extension legs 134. Through such an extension-leg connector, a corresponding extension leg and the extension-leg lumen thereof can be connected to another medical device and a lumen thereof. However, in at least the ready-to-deploy state of the RICC insertion assembly 100 at least one extension-leg connector (e.g., the extension-leg connector including part of the primary lumen 146 of the RICC 102) is connected to the extension-arm clip of the extension arm of the coupler 108 to enforce the loop in the access guidewire 106 and the RICC 102 thereover.

As shown, the RICC 102 is a triluminal RICC including a set of three lumens; however, the RICC 102 is not limited to the set of the three lumens as set forth above. The set of three lumens includes a primary lumen 146, a secondary lumen 148, and a tertiary lumen 150 formed of fluidly connected portions of three catheter-tube lumens, three catheter-hub lumens, and three extension-leg lumens. The primary lumen 146 has a primary-lumen aperture 152 in the distal end of the first section 138 of the catheter tube 130, which corresponds to the distal end of the catheter tube 130 and a distal end of the RICC 102. The secondary lumen 148 has a secondary-lumen aperture 154 in a side of the distal portion of the catheter tube 130. The tertiary lumen 150 has a tertiary-lumen aperture 156 in the side of the distal portion of the catheter tube 130 proximal of the secondary-lumen aperture 154.

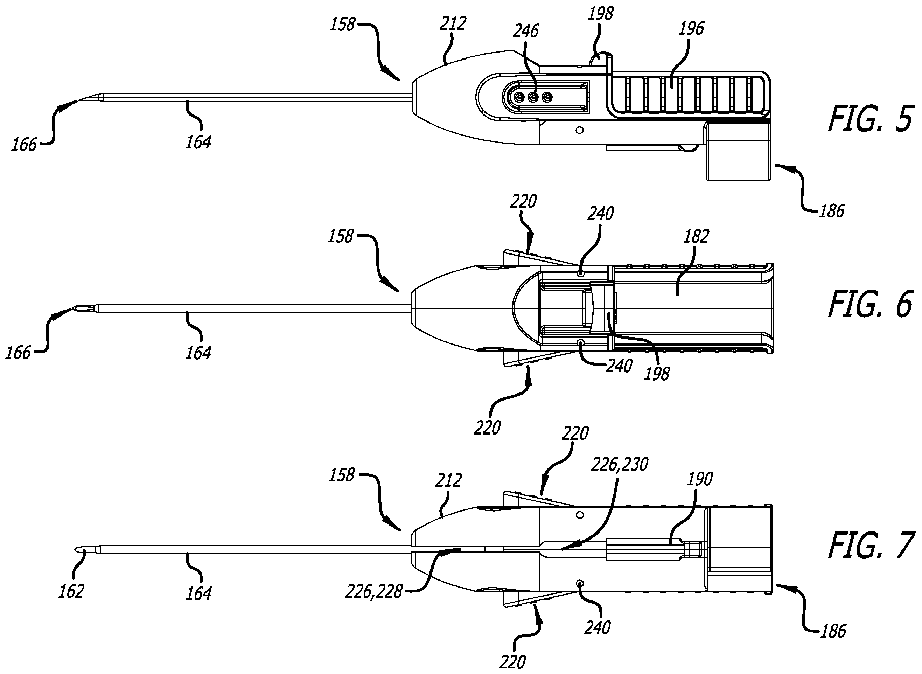

FIGS. 3-12 illustrate various views of an introducer-needle assembly 158 in accordance with some embodiments.

As shown, the introducer-needle assembly 158 is a subassembly of the RICC insertion assembly 100. Indeed, the introducer-needle assembly 158 includes the introducer needle 104 and the coupler 108 coupled together.

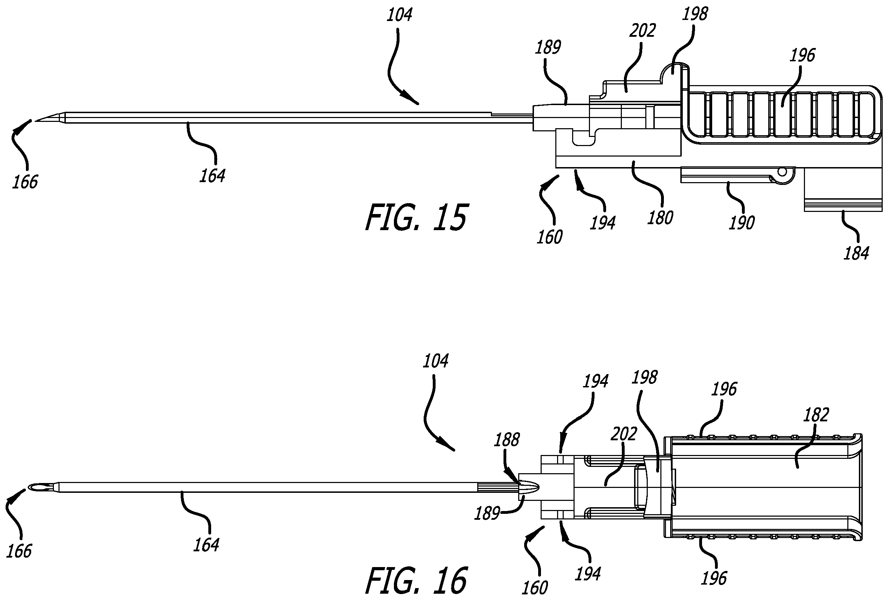

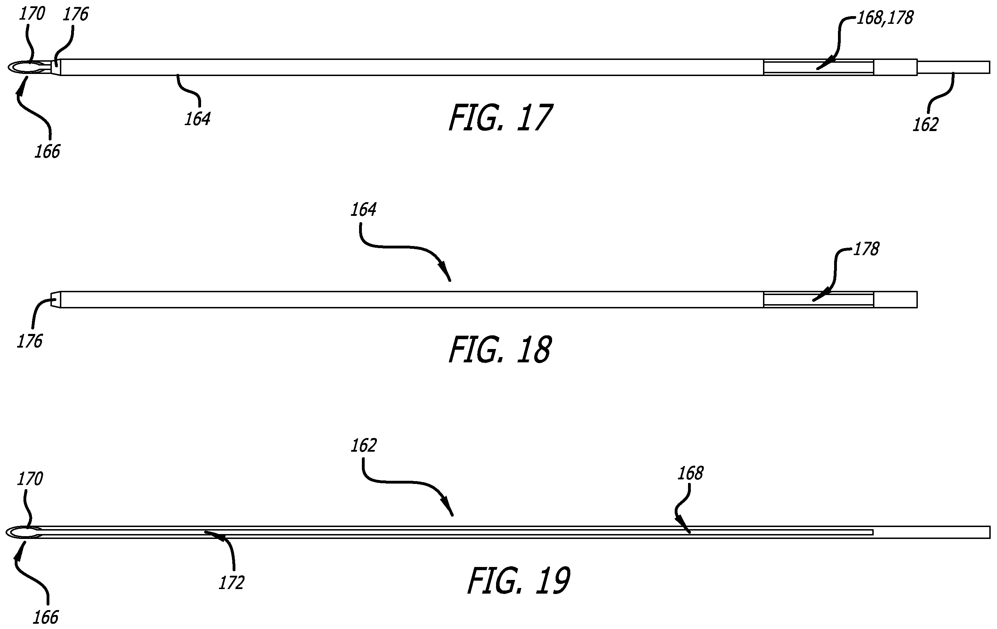

FIGS. 15-19 illustrate various views of the introducer needle 104 or components thereof in accordance with some embodiments.

As shown, the introducer needle 104 includes an elongate portion and a needle hub 160 over a proximal portion of the elongate portion of the introducer needle 104. The elongate portion of the introducer needle 104 includes a needle shaft 162 and a sheath 164 over the needle shaft 162 such that the needle hub 160 is over both a proximal portion of the needle shaft 162 and a proximal portion of the sheath 164. In at least the ready-to-deploy state of the RICC insertion assembly 100, the needle shaft 162 and the sheath 164 thereover extend from the needle hub 160, through the valve module 218 of the coupler 108, and out a distal end of the coupler housing 212 of the coupler 108.

The needle shaft 162 includes a needle tip 166 in a distal portion of the needle shaft 162 and a longitudinal needle slot 168 extending from at least the proximal portion of the needle shaft 162 through the needle tip 166.

The needle tip 166 includes a bevel 170 having a tip bevel and a primary bevel proximal of the tip bevel. While not shown, a tip-bevel angle of the tip bevel is greater than a primary-bevel angle of the primary bevel such that the bevel 170 provides a smooth transition over the needle tip 166. Such a needle tip is thusly configured for establishing a needle tract from an area of skin into a blood-vessel lumen of a patient in accordance with the needle tract-establishing step of the method set forth below.

The needle slot 168 extends from at least the proximal portion of the needle shaft 162 through the needle tip 166. Indeed, the needle slot 168 optionally extends through both a proximal end of the needle shaft 162 and the needle tip 166. Regardless, a needle channel 172 extends along a length of the needle shaft 162 commensurate with the needle slot 168. The needle slot 168 has a width sized in accordance with an outer diameter of the access guidewire 106, which allows the access guidewire 106 to pass from at least the proximal portion of the needle shaft 162 through the needle tip 166 when the introducer needle-withdrawing step of the method set forth below is performed.

While the needle shaft 162 includes the needle slot 168, it should be understood the introducer needle 104 includes a needle lumen 174; however, the needle lumen 174 results from the combination of the needle shaft 162 and the sheath 164 over the needle shaft 162. Indeed, the sheath 164 over the needle shaft 162 seals the needle slot 168 thereunder forming the needle lumen 174 of the introducer needle 104 and enabling the syringe 110 to aspirate blood in accordance with the blood-aspirating step of the method set forth below.

The sheath 164 includes a sheath tip 176 in a distal portion of the sheath 164 and a sheath opening 178 in a side of the proximal portion of the sheath 164.

The sheath tip 176 includes a relatively short taper from an outer diameter of the distal portion of the sheath 164 to an outer diameter of a distal end of the sheath 164, the latter of which is commensurate with an outer diameter of the distal portion of the needle shaft 162. The taper has a taper angle less than the primary-bevel angle of the primary bevel of the needle tip 166, which, in turn, is less than the tip-bevel angle of the tip bevel of the needle tip 166. The sheath tip 176 having such a taper is configured to provide a smooth transition from the needle tip 166 to the sheath body for the needle tract-establishing step of the method set forth below.

The sheath opening 178 opens to the needle slot 168 of the needle shaft 162 allowing the access guidewire 106 to pass through the sheath opening 178 and into the needle channel 172 or the needle lumen 174 formed therefrom in at least the ready-to-deploy state of the RICC insertion assembly 100. Thus, the sheath opening 178 has a width approximately commensurate with a width of the needle slot 168, which, in turn, is sized in accordance with the diameter of the access guidewire 106. The sheath opening 178 also has a length sufficient to allow the access guidewire 106 to pass through the sheath opening 178 and into the needle slot 168 or the needle lumen 174 formed therefrom while also accommodating the blade 236 of the valve module 218 under a distal end of the sheath opening 178. Notably, the sheath 164 over the needle shaft 162 seals the needle slot 168 thereunder except for that under the sheath opening 178. However, the valve module 218 seals over the needle slot 168 exposed by the sheath opening 178 by sealing the proximal portions of the needle shaft 162 and the sheath 164 therein, thereby enabling the syringe 110 to aspirate blood in accordance with the blood-aspirating step of the method set forth below.

The sheath 164, or a sheath body thereof, is formed of a polymeric material configured to facilitate a smooth, consistent insertion of the introducer needle 104 from an area of skin to a blood-vessel lumen of a patient in accordance with the needle tract-establishing step of the method set forth below. In addition, the polymeric material has mechanical properties at a thickness of the sheath 164 sufficient to resist collapse of the sheath 164 into the needle slot 168 of the needle shaft 162 when the blood-aspirating step of the method set forth below is performed, notably, while also facilitating the cutting of the sheath 164 off the needle shaft 162 in accordance with the introducer needle-withdrawing step of the method set forth below. Such a polymeric material can include, but is not limited to, polyethylene, polypropylene, or polytetrafluoroethylene.

The needle hub 160 includes a distally extending needle-hub extension arm 180 in a distal portion of the needle hub 160, a proximally extending needle-hub cradle 182, and a needle-hub portion 184 of a RICC clip 186 connected or suspended from a proximal portion of the needle hub 160, specifically, a proximal portion of the needle-hub cradle 182. In addition, the needle hub 160 can further include an access-guidewire channel 188, a holding means for holding the access guidewire 106 in place, as needed, and a needle-hub alignment rail 190.

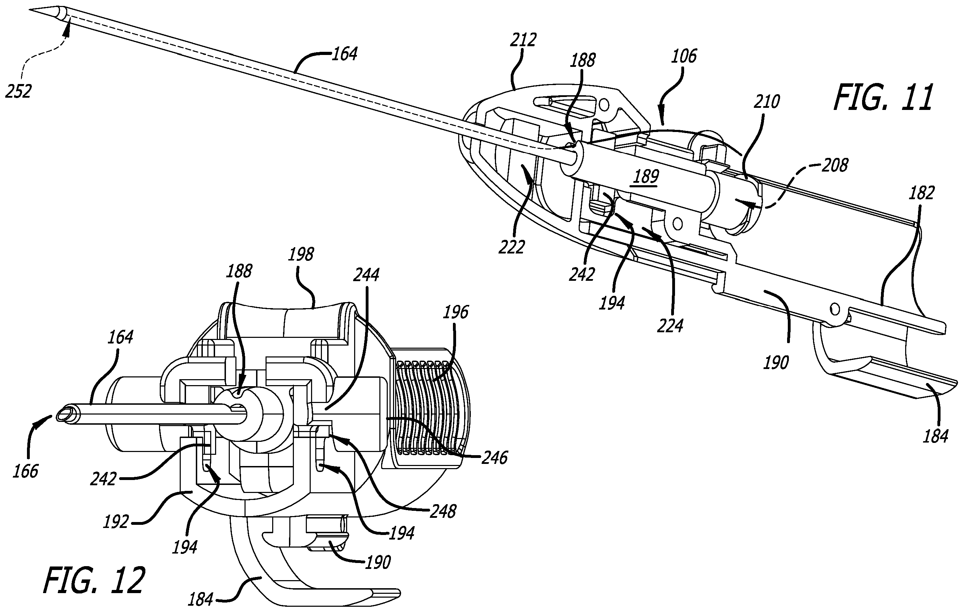

The needle-hub extension arm 180 is configured to be locked in the needle-hub receptacle 224 in at least the ready-to-deploy state of the RICC insertion assembly 100. The needle-hub extension arm 180 includes one or more walls 192 depending upon a configuration of the needle-hub extension arm 180. In an example, the needle-hub extension arm 180 can include one wall when the needle-hub extension arm 180 is configured such that it approximates a half right circular or elliptic cylindrical surface. In another example, the needle-hub extension arm 180 can include three walls when the needle-hub extension arm 180 is configured such that it approximates a sluice box. Such an example of the needle-hub extension arm 180 is shown in FIG. 12, wherein the three walls include a bottom wall and two side walls flanking the bottom wall. Notably, the bottom wall can be curved; however, the bottom wall can alternatively be flat. Regardless of a number of the one-or-more walls 192, the needle-hub extension arm 180 includes up to a pair of notches 194 in the one-or-more walls 192 thereof. As shown in FIG. 12, for example, the needle-hub extension arm 180 can include the pair of notches 194 on opposite sides of the needle-hub extension arm 180 such as in the two side walls of the needle-hub extension arm 180 flanking the bottom wall. The protrusion 242 of each lock button of the pair of lock buttons 220 set forth below is disposed in a corresponding notch of the pair of notches 194 in the needle-hub extension arm 180 in the default state of the pair of lock buttons 220. Thus, the needle-hub extension arm 180, which is disposed in the needle-hub receptacle 224 of the coupler housing 212 in at least the ready-to-deploy state of the RICC insertion assembly 100, is also locked in the needle-hub receptacle 224 in at least the ready-to-deploy state of the RICC insertion assembly 100.

The needle-hub cradle 182 is configured to cradle the barrel 126 of the syringe 110 and, thereby, support the syringe 110 in at least the ready-to-deploy state of the RICC insertion assembly 100. Advantageously, the needle-hub cradle 182 includes optionally textured grip pads 196 on opposite sides of the needle-hub cradle 182 configured to facilitate holding the RICC insertion assembly 100 thereby when establishing a needle tract from an area of skin into a blood-vessel lumen of a patient in accordance with the needle tract-establishing step of the method set forth below. In addition, the grip pads 196 are also configured to facilitate holding the needle hub 160 thereby when withdrawing the introducer needle 104 from the coupler 108 in the introducer needle-withdrawing step of the method set forth below. When the grip pads 196 are textured, the grip pads 196 can be textured with, for example, ridges, bumps, or, inversely, dimples, which facilitate holding the RICC insertion assembly 100 or the needle hub 160 thereof in an environment in which stray fluids can make holding the RICC insertion assembly 100 or the needle hub 160 thereof difficult.

The RICC clip 186 is configured to hold a portion of the RICC 102 therein in at least the ready-to-deploy state of the RICC insertion assembly 100. It follows that the needle-hub portion 184 of the RICC clip 186, too, is configured to hold the foregoing portion of the RICC 102 therein in at least the ready-to-deploy state of the RICC insertion assembly 100. Where the coupler portion 216 of the RICC clip 186 is the first 'C'-shaped portion of the RICC clip 186 as set forth below, the needle-hub portion 184 of the RICC clip 186 is a second 'C'-shaped portion of the RICC clip 186 suspended from the proximal portion of the needle hub 160, specifically, the proximal portion of the needle-hub cradle 182. The first and second 'C'-shaped portions of the RICC clip 186 open toward each other such that the RICC clip 186 is completed, optionally, overlappingly completed, in at least the ready-to-deploy state of the RICC insertion assembly 100. When completed in at least the ready-to-deploy state of the RICC insertion assembly 100, the RICC clip 186 can hold, for example, the one-or-more extension legs 134 of the RICC 102 therein such that the RICC 102 is suspended from the RICC insertion assembly 100 as shown in FIG. 1. Notably, the RICC clip 186 is configured to release the portion (e.g., the one-or-more extension legs 134) of the RICC 102 held therein when the introducer needle 104 is withdrawn from the coupler 108 in the introducer needle-withdrawing step of the method set forth below. Indeed, withdrawing the introducer needle 104 from the coupler 108 separates the coupler and needle-hub portions 216 and 184 of the RICC clip 186, thereby opening the RICC clip 186 to release the portion (e.g., the one-or-more extension legs 134) of the RICC 102 held therein.

The access-guidewire channel 188 is configured to guide the distal portion of the access guidewire 106 from the distal end of the RICC 102 and into the valve module 218 by way of the access-guidewire conduit 234 in at least the ready-to-deploy state of the RICC insertion assembly 100. As shown, the access-guidewire channel 188 is defined by a notch in a distal portion of a needle-hub extension tube 189, which portion of the needle-hub extension tube 189 is over the proximal portions of the needle shaft 162 and the sheath 164 just proximal of or slightly over the sheath opening 178 of the sheath 164. Notably, the access-guidewire channel 188 can alternatively be configured as another access-guidewire conduit but through the distal portion of the needle-hub extension tube 189.

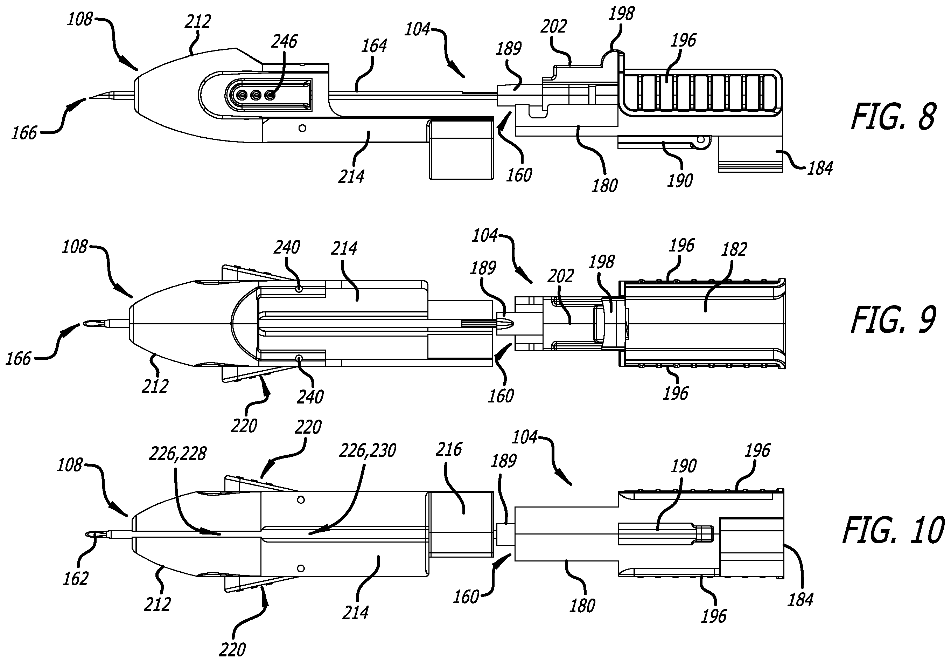

The holding means for holding the access guidewire 106 in place, as needed, can include a transverse bar 198 and a needle-hub pad 202 between the bar 198 and the access-guidewire channel 188 as best shown in FIGS. 8, 9, 11 and 12. Notably, both the bar 198 and the needle-hub pad 202 are also between sides of the coupler housing 212 in at least the ready-to-deploy state of the RICC insertion assembly 100. The bar 198 and the needle-hub pad 202 are configured for pressing and holding (e.g., with a thumb) the access guidewire 106 in place thereagainst, as needed, such as when establishing a needle tract from an area of skin to a blood-vessel lumen of a patient. However, the holding means for holding the access guidewire 106 in place, as needed, is not limited to the bar 198 and the needle-hub pad 202, which are for manually holding the guidewire in place. Indeed, the holding means for holding the access guidewire 106 in place, as needed, can alternatively be a pinch holder 204 as shown in FIG. 2. Such a pinch holder can include a pair of closely spaced or touching elastomeric right circular solid cylinders 206 oriented longitudinally along the needle hub 160 such that the access guidewire 106 can be pressed into the pinch holder 204 and automatically held therein by compression on the access guidewire 106 by the elastomeric cylinders 206.

The needle-hub alignment rail 190, which extends from the needle-hub cradle 182 on a same side as the needle-hub portion 184 of the RICC clip 186, albeit distal thereto, is configured for at least aligning the needle hub 160 with the coupler 108 when inserting the distal portion of the needle hub 160 into the needle-hub receptacle 224 of the coupler housing 212 while assembling the RICC insertion assembly 100 or the introducer-needle assembly 158 thereof. Notably, as set forth below, the coupler-housing slot 226 of the coupler housing 212 includes the wider portion 230 of the coupler-housing slot 226 configured to accept the needle-hub alignment rail 190 therein for aligning the needle hub 160 with the coupler 108 while assembling the RICC insertion assembly 100 or the introducer-needle assembly 158 thereof. Advantageously, the needle-hub alignment rail 190 disposed in the wider portion 230 of the coupler-housing slot 226 minimizes or eliminates any relative rotation and, thus, any misalignment, between the needle hub 160 and the coupler 108, thereby ensuring the needle-hub extension arm 180 of the needle hub 160 can be locked in the needle-hub receptacle 224 of the coupler 108 and unlocked therefrom with, for example, the pair of lock buttons 220 without incident (e.g., jamming).

The needle-hub connector also includes a needle-hub bore 208 and an optional needle-hub flange 210 about the needle-hub bore 208.

The needle-hub bore 208 of the needle-hub connector is configured to accept a syringe tip (not shown) of the syringe 110 therein for fluidly connecting the introducer needle 104 to the syringe 110. (*See* FIGS. 1-4 for the fluidly connected introducer needle 104 and syringe 110.) Indeed, the needle-hub bore 208 can have a Luer taper (e.g., a 6% taper) configured to accept the syringe tip therein, which syringe tip can be complementarily configured with a Luer taper.

When present, the needle-hub flange 210 about the needle-hub bore 208 is configured to screw together with internal threads of a threaded collar around the syringe tip of the syringe 110. While the threaded collar of the syringe 110 is also optional, the needle-hub flange 210 advantageously provides a so-called Luer lock-style connection with the internal threads of the threaded collar when both are present. This provides added security against inadvertent disconnection of the introducer needle 104 and the syringe 110 over that provided by an otherwise Luer slip-style connection.

FIGS. 8-10 illustrate various views of the coupler 108 in accordance with some embodiments.

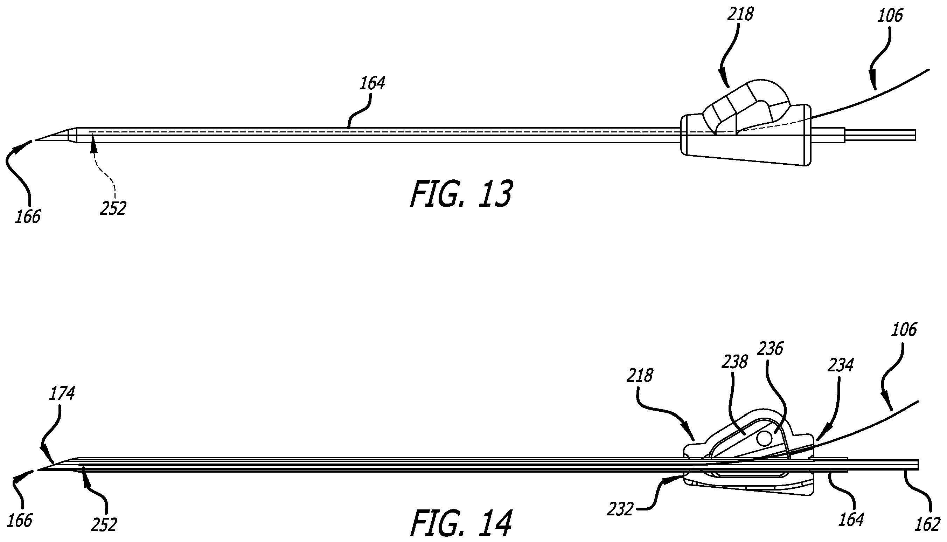

As shown, the coupler 108 includes a coupler housing 212, a proximally extending coupler-housing support 214 of the coupler housing 212, and a coupler portion 216 of the RICC clip 186 connected or suspended from a proximal portion of the coupler 108, specifically, a proximal portion of the coupler-housing support 214. While not shown in FIGS. 8-10, the coupler 108 further includes a valve module 218 disposed in the coupler housing 212, specifically, the valve-module compartment 222 of the coupler housing 212 distal of the needle-hub receptacle 224. Such a valve module is instead shown in FIGS. 13 and 14 over the sheath opening 178 of the sheath 164 of the introducer needle 104. Lastly, the coupler 108 can further include up to a pair of lock buttons 220 extending through opposite sides of the coupler housing 212. That said, it should be understood the coupler 108 can alternatively include a single lock button extending through a side of the coupler housing 212 in some embodiments.

FIG. 11 illustrates a perspective view of the introducer-needle assembly 158 in accordance with some embodiments, wherein half of the coupler housing 212 is removed to assist in visualizing certain features thereof.

The coupler housing 212 includes two molded pieces coupled (e.g., fastened or screwed with screws or bolts) together to form a bullet-shaped body configured to be comfortably held underhand (e.g., cradled) in either a left hand for a left-handed venipuncture or a right hand for a right-handed venipuncture with the RICC insertion assembly 100. An inside of each piece of the two molded pieces includes depressions that form a valve-module compartment 222 in a distal portion of the coupler housing 212 and a needle-hub receptacle 224 distal thereof in a proximal portion of the coupler housing 212 when the two molded pieces are coupled together. While not expressly shown, each piece of the two molded pieces also includes a lock-button through hole configured to accommodate a lock button of the pair of lock buttons 220 that extend through opposite sides of the coupler housing 212 as set forth below. That said, only a single lock-button through hole is needed between the two molded pieces of the coupler housing 212 in embodiments of the introducer-needle assembly 158 utilizing a single lock button to lock the needle hub 160 in the coupler 108 instead of the pair of lock buttons 220. Lastly, the coupler housing 212 includes a longitudinal coupler-housing slot 226 formed between the two molded pieces when coupled together.

The valve-module compartment 222 is configured to hold the valve module 218 therein. Notably, the valve-module compartment 222 is further configured with sufficient space to allow the valve module 218 to separate for the escape of the access guidewire 106 therefrom when the introducer needle 104 is withdrawn from the coupler 108 in the introducer needle-withdrawing step of the method set forth below.

The needle-hub receptacle 224 is configured to accept both the needle-hub extension tube 189 and the needle-hub extension arm 180 of the needle hub 160 when the distal portion of the needle hub 160 of the introducer needle 104 is inserted therein. Indeed, the needle-hub receptacle 224 includes the needle-hub extension tube 189 and the needle-hub extension arm 180 inserted therein in at least the ready-to-deploy state of the RICC insertion assembly 100 or the introducer-needle assembly 158 thereof. Notably, the needle-hub extension arm 180 of the needle hub 160 with the pair of notches 194 in the one-or-more walls 192 of the needle-hub extension arm 180 is configured to be locked in the needle-hub receptacle 224 by the pair of lock buttons 220 in at least the ready-to-deploy state of the RICC insertion assembly 100 or the introducer-needle assembly 158 thereof.

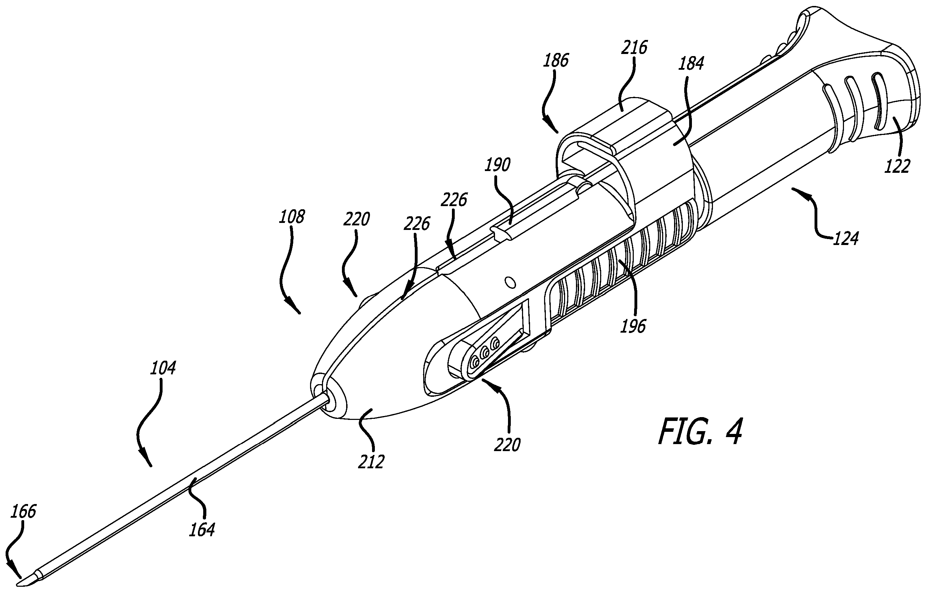

The coupler-housing slot 226 is configured to allow the access guidewire 106 to escape from the coupler housing 212 when the introducer needle 104 is withdrawn from the coupler 108 in the introducer needle-withdrawing step of the method set forth below. The coupler-housing slot 226 is formed on a same side of the coupler housing 212 as the coupler portion 216 of the RICC clip 186 when the two molded pieces of the coupler housing 212 are coupled together. In addition, the coupler-housing slot 226 opens in a direction opposite that of the needle slot 168 of needle shaft 162. The coupler-housing slot 226 is thusly configured to allow the access guidewire 106 to drop from the coupler housing 212 to escape therefrom as the introducer needle 104 is withdrawn from the coupler 108 in the introducer needle-withdrawing step of the method set forth below. Notwithstanding the foregoing, it should be understood the coupler-housing slot 226 can be alternatively located in another location than that set forth above and illustrated in FIGS. 4, 7, and 10. For example, the coupler-housing slot 226 can be alternatively located opposite or orthogonal to that set forth above and illustrated in FIGS. 4, 7, and 10, notably, with other modifications such as relocating the pair of lock-button through holes.

The coupler-housing slot 226 is also configured for at least aligning the needle hub 160 with the coupler 108 when inserting the distal portion (e.g., the needle-hub extension arm 180) of the needle hub 160 into the needle-hub receptacle 224 of the coupler housing 212 while assembling the RICC insertion assembly 100 or the introducer-needle assembly 158 thereof. As best shown in FIG. 10, the coupler-housing slot 226 has a narrower portion 228 and a wider portion 230 proximal of the narrower portion 228, wherein the wider portion 230 of the coupler-housing slot 226 is in the coupler-housing support 214. The wider portion 230 of the coupler-housing slot 226 is configured to accommodate the needle-hub alignment rail 190 of the needle hub 160 for at least aligning the needle hub 160 with the coupler 108 while assembling the RICC insertion assembly 100. Advantageously, the wider portion 230 of the coupler-housing slot 226 minimizes or eliminates any relative rotation and, thus, any misalignment, between the needle hub 160 and the coupler 108 when the needle-hub alignment rail 190 is disposed therein, thereby ensuring the needle-hub extension arm 180 of the needle hub 160 can be locked in the needle-hub receptacle 224 of the coupler 108 and unlocked therefrom with, for example, the pair of lock buttons 220 without incident (e.g., jamming).

The coupler-housing support 214 is configured to support the introducer needle 104 by way of the needle-hub extension arm 180 and the needle-hub cradle 182 of the needle hub 160 when the introducer needle 104 is coupled with the coupler 108. Notably, at least the needle-hub extension arm 180 of the needle hub 160 is slidably disposed over the coupler-housing support 214 in at least the ready-to-deploy state of the RICC insertion assembly 100. Indeed, the needle-hub cradle 182 of the needle hub 160 is also slidably disposed over the coupler-housing support 214 in at least the ready-to-deploy state of the RICC insertion assembly 100; however, the needle-hub alignment rail 190 extending from the needle-hub cradle 182 is also slidably disposed in the wider portion 230 of the coupler-housing slot 226 in the coupler-housing support 214.

Again, the RICC clip 186 is configured to hold a portion of the RICC 102 therein in at least the ready-to-deploy state of the RICC insertion assembly 100. It follows that the coupler portion 216 of the RICC clip 186, too, is configured to hold the foregoing portion of the RICC 102 therein in at least the ready-to-deploy state of the RICC insertion assembly 100. The coupler portion 216 of the RICC clip 186 is a first 'C'-shaped portion of the RICC clip 186 suspended from a proximal portion of the coupler-housing support 214, whereas the needle-hub portion 184 of the RICC clip 186 is the second 'C'-shaped portion of the RICC clip 186 as set forth above. Again, the first and second 'C'-shaped portions of the RICC clip 186 open toward each other such that the RICC clip 186 is completed, optionally, overlappingly completed, in at least the ready-to-deploy state of the RICC insertion assembly 100. When completed in at least the ready-to-deploy state of the RICC insertion assembly 100, the RICC clip 186 can hold, for example, the one-or-more extension legs 134 of the RICC 102 therein such that the RICC 102 is suspended from the RICC insertion assembly 100 as shown in FIG. 1. Notably, the RICC clip 186 is configured to release the portion (e.g., the one-or-more extension legs 134) of the RICC 102 held therein when the introducer needle 104 is withdrawn from the coupler 108 in the introducer needle-withdrawing step of the method set forth below. Indeed, withdrawing the introducer needle 104 from the coupler 108 separates the coupler and needle-hub portions 216 and 184 of the RICC clip 186, thereby opening the RICC clip 186 to release the portion (e.g., the one-or-more extension legs 134) of the RICC 102 held therein.

FIGS. 13 and 14 illustrate different views of the valve module 218 of the coupler 108 over both the sheath 164 and needle shaft 162 of the introducer needle 104 in accordance with some embodiments.

As shown, the valve module 218 is configured to seal around the proximal portion of the sheath 164 and the distal portion of the access guidewire 106 that extends through the sheath opening 178 in at least the ready-to-deploy state of the RICC insertion assembly 100. Such seals around the sheath 164 and the access guidewire 106 enable leak-free aspiration through the introducer needle 104 during the blood-aspirating step of the method set forth below. The valve module 218 includes an introducer-needle conduit 232, an access-guidewire conduit 234, and an integrated blade 236.

The introducer-needle conduit 232 is configured to direct the elongate portion (i.e., the needle shaft 162 and the sheath 164 thereover) of the introducer needle 104 through both the valve module 218 and the coupler housing 212. Indeed, the introducer-needle conduit 232 includes the elongate portion of the introducer needle 104 disposed therein in at least the ready-to-deploy state of the RICC insertion assembly 100 or the introducer-needle assembly 158 thereof. While not shown, the introducer-needle conduit 232 additionally includes a seal (e.g., two halves of an elastomeric sleeve) therein configured to seal around the elongate portion of the introducer needle 104 when the introducer needle 104 is disposed and, optionally, compressed in the introducer-needle conduit 232.

The access-guidewire conduit 234 is configured to direct the access guidewire 106 from the coupler-housing slot 226 of the coupler housing 212 into both the sheath opening 178 of the sheath 164 and the needle channel 172 of the needle shaft 162 or the needle lumen 174 of the introducer needle 104. Indeed, the access-guidewire conduit 234 includes the access guidewire 106 disposed therein in at least the ready-to-deploy state of the RICC insertion assembly 100. While not shown, the access-guidewire conduit 234 additionally includes a seal (e.g., two halves of an elastomeric sleeve) therein configured to seal around the distal portion of the access guidewire 106 when the access guidewire 106 is disposed and, optionally, compressed in the access-guidewire conduit 234.

The blade 236 extends from an attachment point in the valve module 218 and into the needle slot 168 of the needle shaft 162 such that the blade 236 is disposed in the needle slot 168 under the distal end of the sheath opening 178 of the sheath 164. The blade 236 includes a distal-facing blade edge 238 configured to cut the sheath 164 off the needle shaft 162 as the introducer needle 104 is withdrawn in a proximal direction from the coupler 108 in the introducer needle-withdrawing step of the method set forth below. Cutting the sheath 164 off the needle shaft 162 allows the access guidewire 106 to escape from both the needle shaft 162 by way of the needle slot 168 thereof and the coupler 108 by way of the coupler-housing slot 226 of the coupler housing 212.

The pair of lock buttons 220 respectively extending through the pair of lock-button through holes on opposite sides of the coupler housing 212 are configured to lock the needle hub 160 of the introducer needle 104 in the coupler 108. Specifically, the pair of lock buttons 220 are configured to lock the needle-hub extension arm 180 of the needle hub 160 in the needle-hub receptacle 224 of the coupler 108. For this, the pair of lock buttons 220 are respectively mounted on a pair of axles 240 captively held in the coupler housing 212 such that each lock button of the pair of lock buttons 220 pivots on a corresponding axle. Notably, the pair of axles 240 can be through proximal portions of the pair of lock buttons 220, as shown, or the pair of axles 240 can be relocated in the coupler housing 212 such that the pair of axles 240 are through distal portions of the pair of lock buttons 220. Each lock button of the pair of lock buttons 220 has a protrusion 242 extending from a crossarm 244 connecting the protrusion 242 to an external, pressable portion 246 of the lock button. In addition, each lock button of the pair of lock buttons 220 has a gap 248 opposite the crossarm 244 between the protrusion 242 and the external, pressable portion 246 of the lock button. Lastly, while not shown, the pair of lock buttons 220 can be biased away from a centerline of the coupler 108 with a pair of compression springs, respectively, or a single compression spring between the pair of lock buttons 220. Regardless, the pair of lock buttons 220 can be biased such that the protrusion 242 of each lock button of the pair of lock buttons 220 is disposed in a notch of the pair of notches 194 in the one-or-more walls 192 of the needle-hub extension arm 180 on opposite sides of the needle-hub extension arm 180 in a default state of the pair of lock buttons 220. However, the protrusion 242 of each lock button of the pair of lock buttons 220 is displaced from the foregoing when the pair of lock buttons 220 are pressed into the coupler housing 212. Indeed, when the pair of lock buttons 220 are pressed into the coupler housing 212, the one-or-more walls 192 of the needle-hub extension arm 180 on both sides of the needle-hub extension arm 180 can pass through the gap 248 between the protrusion 242 and the external, pressable portion 246 of each lock button of the pair of lock buttons 220, thereby allowing the introducer needle 104 to be withdrawn from the coupler 108 in the introducer needle-withdrawing step of the method set forth below.

FIGS. 1 and 2 illustrate the access guidewire 106 as part of the RICC insertion assemblies 100 and 200 in accordance with some embodiments.

The access guidewire 106 includes a proximal portion including a proximal end, optionally, disposed in an access-guidewire hub 250, and a distal portion including a distal end. In at least the ready-to-deploy state of the RICC insertion assembly 100, the proximal end of the access guidewire 106 extends from a proximal end of the RICC 102, optionally, within the access-guidewire hub 250 coupled to a Luer connector of the RICC 102. Regardless, the proximal portion of the access guidewire 106 extends from the proximal end of the RICC 102, through the foregoing Luer connector, and along the primary lumen 146 of the RICC 102 in at least the ready-to-deploy state of the RICC insertion assembly 100. The distal portion of the access guidewire 106 also extends along the primary lumen 146 of the RICC 102, but the distal portion of the access guidewire 106 further extends out the distal end of the RICC 102, into the valve module 218 by way of the access-guidewire conduit 234, into the needle shaft 162 through both the sheath opening 178 of the sheath 164 and the needle slot 168 of the needle shaft 162, and along the needle channel 172 of the needle shaft 162 or the needle lumen 174 of the introducer needle 104 in the ready-to-deploy state of the RICC insertion assembly 100. As shown in FIGS. 1 and 2, the distal end of the access guidewire 106 is disposed in the needle lumen 174 of the introducer needle 104 just proximal of the needle tip 166 in at least the ready-to-deploy state of the RICC insertion assembly 100. Again, the proximal and distal ends of the access guidewire 106 enforce the loop in the access guidewire 106 in at least the ready-to-deploy state of the RICC insertion assembly 100, which loop the RICC 102 is disposed over, thereby keeping the RICC insertion assembly 100 in a relatively compact form.

The access-guidewire hub 250 over the distal end of the access guidewire 106 is configured to provide a convenient handle by which the access guidewire 106 can be withdrawn from the RICC insertion assembly 100 or subassembly thereof during the access guidewire-withdrawing step set forth below. In addition, the access-guidewire hub 250 advantageously prevents over advancement of the access guidewire 106 during the access guidewire-advancing step of the method set forth below, which, in turn, obviates losing the proximal end of the access guidewire 106 in the primary lumen 146 of the RICC 102. While the access-guidewire hub 250 can be any convenient shape, the access-guidewire hub 250 of FIGS. 1 and 2 is shown as a tab having relatively flat major surfaces. While flat, such major surfaces can be textured with, for example, ridges, bumps, or, inversely, dimples, which facilitate holding the access-guidewire hub 250 in an environment in which stray fluids can make holding the access-guidewire hub 250 difficult.

The access guidewire 106 can include a guidewire tip 252 in the distal portion of the access guidewire 106, which adopts a 'J' shape configured to prevent puncturing a back wall of a blood vessel. Such a guidewire tip assumes a straightened state in at least the ready-to-deploy state of the RICC insertion assembly 100 and a curved state when the guidewire tip 252 is advanced beyond the needle tip 166 (e.g., advanced into a blood-vessel lumen) in a deployed state of the RICC insertion assembly 100.

The access guidewire 106 can further include a bare-wire portion and a wound-wire portion proximal of the bare-wire portion. While not shown, the bare-wire portion, when present, distally extends through the access-guidewire conduit 234 of the valve module 218 in at least the ready-to-deploy state of the RICC insertion assembly 100 such that the valve module 218 forms a fluid-tight seal around the bare-wire portion of the access guidewire 106. Notably, the foregoing bare-wire portion can instead be a flat-wound or ground-wound portion of the access guidewire 106, wherein the flat-wound portion includes windings of a tape instead of a round wire, and wherein the ground-wound portion includes windings of a round wire ground down to flatten the windings.

### Methods (not claimed)

Methods include a method for inserting the RICC 102 into a blood-vessel lumen of a patient. Such a method includes one or more steps selected from a RICC insertion assembly-obtaining step, a needle tract-establishing step, a blood-aspirating step, an access guidewire-advancing step, a needle hub-unlocking step, an introducer needle-withdrawing step, a RICC-advancing step, an access guidewire-withdrawing step, a maneuver guidewire-advancing step, another RICC-advancing step, and a maneuver guidewire-withdrawing step.

The RICC insertion assembly-obtaining step includes obtaining the RICC insertion assembly 100. As set forth above, the RICC insertion assembly 100 includes the RICC 102, the introducer needle 104 including the sheath 164 over the needle shaft 162, and the access guidewire 106 coupled together by the coupler 108. In addition, the proximal portion of the access guidewire 106 is disposed in the primary lumen 146 of the RICC 102 and the distal end of the access guidewire 106 is disposed in the introducer needle 104 by way of the valve module 218 disposed in the coupler housing 212 of the coupler 108. The proximal and distal ends of the access guidewire 106 enforce the loop in the access guidewire 106 over which the RICC 102 is disposed in at least the ready-to-deploy state of the RICC insertion assembly 100.

The needle tract-establishing step includes establishing a needle tract from an area of skin to the blood-vessel lumen with the introducer needle 104. In addition, the needle tract-establishing step can include pressing and holding the access guidewire 106 in place against the bar 198, the needle-hub pad 202 of the needle hub 160, or both during the establishing of the needle tract. Again, the needle-hub pad 202 is between the bar 198 and the access-guidewire channel 188 defined by the notch in the distally extending needle-hub extension tube 189. Lastly, the needle tract-establishing step can include ensuring blood flashback while establishing the needle tract. Ensuring blood flashback while establishing the needle tract includes ensuring blood flashes back into the needle hub 160 of the introducer needle 104, particularly when the needle hub 160 is clear and colorless, the syringe tip of the syringe 110 fluidly connected to the introducer needle 104, the barrel 126 of the syringe 110, or a combination thereof. A slight vacuum can be drawn with the syringe 110 while establishing the needle tract such that the blood flashes back into at least the needle hub 160 of the introducer needle 104 upon establishing the needle tract. Ensuring the blood flashes back in accordance with the foregoing confirms the needle tract extends into the blood-vessel lumen.

The blood-aspirating step includes aspirating blood with the syringe 110 coupled to the needle hub 160 for confirmation the needle tract extends into the blood-vessel lumen, notably before withdrawing the introducer needle 104 from the coupler 108 in the introducer needle-withdrawing step. Again, the sheath 164 over the needle shaft 162 seals the needle slot 168 of the needle shaft 162 thereunder. In particular, the sheath 164 seals the needle slot 168 outside of the valve module 218. The valve module 218, in turn, seals over the sheath opening 178 of the sheath 164, which sheath opening 178 allows the access guidewire 106 to pass into the needle shaft 162 by way of the needle slot 168 in the ready-to-deploy state of the RICC insertion assembly 100. The valve module 218 also seals around the distal portion of the access guidewire 106. Such seals enable the syringe 110 to aspirate blood in the blood-aspirating step.

The access guidewire-advancing step includes advancing the distal end of the access guidewire 106 from its initial location in the needle shaft 162 just proximal of the needle tip 166 of the needle shaft 162 into the blood-vessel lumen, thereby securing blood-vessel access for the RICC 102 in the RICC-advancing step.

The needle hub-unlocking step includes unlocking the needle hub 160 before the withdrawing of the introducer needle 104 from the coupler 108 in the introducer needle-withdrawing step. The unlocking of the needle hub 160 includes pressing the pair of lock buttons 220 extending through the opposite sides of the coupler housing 212 into the coupler housing 212, thereby allowing the one-or-more walls 192 of the distally extending needle-hub extension arm 180 to pass through the gap 248 between the protrusion 242 and the external, pressable portion 246 of each lock button of the pair of lock buttons 220.

The introducer needle-withdrawing step includes withdrawing the introducer needle 104 by the needle hub 160 from the coupler 108 leaving the access guidewire 106 in place in the blood-vessel lumen. The introducer needle-withdrawing step includes simultaneously cutting the sheath 164 off the needle shaft 162 with the blade 236 of the valve module 218 disposed in the valve-module compartment 222 of the coupler housing 212 when the introducer needle 104 is withdrawn from the coupler 108. The cutting of the sheath 164 off the needle shaft 162 allows the access guidewire 106 to escape from the needle shaft 162 by way of the needle slot 168 thereof. Notably, the valve module 218 around the needle shaft 162 and the sheath 164 separates to allow the access guidewire 106 to further escape from the valve module 218 when the introducer needle 104 is withdrawn from the coupler 108 in the introducer needle-withdrawing step. In addition, the coupler housing 212 includes the coupler-housing slot 226 configured to allow the access guidewire 106 to yet further escape from the coupler housing 212 when the introducer needle 104 is withdrawn from the coupler 108 in the introducer needle-withdrawing step. Lastly, the introducer needle-withdrawing step includes simultaneously separating the RICC clip 186 formed between the needle-hub portion 184 and the coupler portion 216 of the RICC clip 186. Indeed, the RICC clip 186 separates to allow the portion (e.g., the one-or-more extension legs 134) of the RICC 102 held therein to escape with the withdrawing of the introducer needle 104 from the coupler 108.

The RICC-advancing step includes advancing the catheter tube 130 of the RICC 102 over the access guidewire 106 and into the blood-vessel lumen, thereby inserting the RICC 102 into the blood-vessel lumen.

The access guidewire-withdrawing step includes withdrawing the access guidewire 106 leaving the catheter tube 130 in place in the blood-vessel lumen.

The maneuver guidewire-advancing step includes advancing a maneuver guidewire into the blood-vessel lumen by way of the primary lumen 146 of the RICC 102 and to a lower ⅓ of a superior vena cava ("SVC") of a heart of the patient.

The other RICC-advancing step includes advancing the distal portion of the catheter tube 130 farther into the blood-vessel lumen over the maneuver guidewire to the lower ⅓ of the SVC of the heart of the patient.

The maneuver guidewire-withdrawing step includes withdrawing the maneuver guidewire leaving the catheter tube 130 in place in the lower ⅓ of the SVC.

While some particular embodiments have been disclosed herein, and while the particular embodiments have been disclosed in some detail, it is not the intention for the particular embodiments to limit the scope of the concepts provided herein. Additional adaptations or modifications can appear to those of ordinary skill in the art, and, in broader aspects, these adaptations or modifications are encompassed as well. Accordingly, departures may be made from the particular embodiments disclosed herein without departing from the scope of the concepts provided herein.

## Claims

1. An introducer-needle assembly (158), comprising:
an introducer needle (104) including:
a needle shaft (162) including a longitudinal needle slot (168) extending from a proximal portion of the needle shaft through a distal needle tip (166);
a sheath (164) over the needle shaft (162) sealing the needle slot (168) thereunder except for that under a sheath opening (178) in a proximal portion of the sheath;
a needle hub (160) over the proximal portions of the needle shaft and the sheath, the needle hub including a distally extending needle-hub extension arm (180); and
a needle-hub portion (184) of a rapidly insertable central catheter ("RICC") clip (186) configured to hold a portion of a RICC (102) therein; and
a coupler (108) coupled with the introducer needle, the coupler including:
a coupler housing (212); and
a coupler portion (216) of the RICC clip (186) connected to the coupler housing.

2. The introducer-needle assembly according to claim 1, wherein the coupler housing (212) includes a proximally extending coupler-housing support (214) over which at least the needle-hub extension arm (180) is slidably disposed.

3. The introducer-needle assembly according to claim 2, wherein the coupler portion (216) of the RICC clip (186) is a first 'C'-shaped portion of the RICC clip suspended from a proximal portion of the coupler-housing support (214), the needle-hub portion (184) of the RICC clip (186) is a second 'C'-shaped portion of the RICC clip suspended from a proximal portion of the needle hub (160), and the first and second 'C'-shaped portions of the RICC clip open toward each other completing the RICC clip.

4. The introducer-needle assembly according to any of claims 1-3, wherein the coupler housing (212) includes a needle-hub receptacle (224) in a proximal portion of the coupler housing into which needle-hub receptacle a distal portion of the needle hub (160) including the needle-hub extension arm (180) is disposed.

5. The introducer-needle assembly according to claim 4, the coupler further including a valve module (218) disposed in a valve-module compartment (222) of the coupler housing (212) distal of the needle-hub receptacle (224), the valve module sealing around the proximal portion of the sheath (164) and, when present, a distal portion of an access guidewire (106) that extends through the sheath opening, thereby enabling leak-free aspiration through the introducer needle.

6. The introducer-needle assembly according to claim 5, wherein the valve module (218) includes an integrated blade (236) disposed in the needle slot (168) under a distal end of the sheath opening (178), the blade including a distal-facing blade edge configured to cut the sheath off the needle shaft when the introducer needle is withdrawn from the coupler.

7. The introducer-needle assembly according to any of claims 1-6, wherein the coupler (108) includes a pair of lock buttons (220) extending through opposite sides of the coupler housing (212), the pair of lock buttons respectively mounted on a pair of axles (240) captively held in the coupler housing (212) such that each lock button of the pair of lock buttons pivots on a corresponding axle.

8. The introducer-needle assembly according to claim 7, wherein each lock button of the pair of lock buttons (220) has a protrusion (242) extending from a crossarm (244) connecting the protrusion (242) to an external, pressable portion of the lock button and a gap opposite the crossarm between the protrusion and the external, pressable portion of the lock button.

9. The introducer-needle assembly according to claim 8, wherein the pair of lock buttons (220) are biased away from a centerline of the coupler (108) such that the protrusion (242) of each lock button of the pair of lock buttons (220) is disposed in a notch of a pair of notches (194) in a wall of the needle-hub extension arm (180) on opposite sides of the needle-hub extension arm in a default state of the pair of lock buttons.

10. The introducer-needle assembly according to claim 9, wherein the wall of the needle-hub extension arm (180) on both sides of the needle-hub extension arm passes through the gap between the protrusion (242) and the external, pressable portion of each lock button of the pair of lock buttons (220) when the pair of lock buttons are pressed in toward the centerline of the coupler.

11. A rapidly insertable central catheter ("RICC") insertion assembly (100), comprising:
a RICC (102);
an introducer needle assembly (158) according to any one of claims 1 to 10,
an access guidewire (106) including:
a proximal portion including a proximal end; and
a distal portion including a distal end disposed in the introducer needle (104) just proximal of the needle tip in at least the ready-to-deploy state of the RICC insertion assembly; and
the coupler (108) coupling the RICC (102), the introducer needle (104), and the access guidewire (106) together, the proximal and distal ends of the access guidewire (106) enforcing a loop in the access guidewire over which the RICC (102) is disposed in at least the ready-to-deploy state of the RICC insertion assembly.

12. The RICC insertion assembly according to claim 11, wherein the needle hub (160) includes a proximally extending needle-hub cradle (182) with grip pads (196) on opposite sides of the needle-hub cradle (182) to facilitate holding the RICC insertion assembly (158) thereby when the introducer needle is withdrawn from the coupler, optionally
further comprising a syringe (110) fluidly coupled to the introducer needle (104) in at least the ready-to-deploy state of the RICC insertion assembly (158), a barrel (126) of the syringe (110) disposed in the needle-hub cradle (182) with the grip pads (196) flanking the barrel of the syringe, further optionally
another RICC clip (112) incorporated into a plunger-flange extension (114) extending from a plunger flange (116) of a plunger (120) of the syringe, the other RICC clip configured to hold a catheter hub (132) of the RICC therein such that the RICC (102) is suspended from the RICC insertion assembly in at least the ready-to-deploy state of the RICC insertion assembly.

13. The RICC insertion assembly according to any of claims 11 or 12, wherein the needle hub (160) includes an access-guidewire channel (188) defined by a notch in a distally extending needle-hub extension tube (189) over the proximal portions of the needle shaft and the sheath, optionally
wherein the needle hub includes a transverse bar (198) and a needle-hub pad (202) between the bar and the access-guidewire channel (188), the bar and the needle-hub pad configured for pressing and holding the access guidewire in place thereagainst when establishing a needle tract from an area of skin to a blood-vessel lumen of a patient.

14. The RICC insertion assembly according to any of claims 11 to 13, wherein the coupler housing (212) includes a longitudinal coupler-housing slot (226) on a same side of the coupler housing as the coupler portion (216) of the RICC clip (186), the coupler-housing slot thereby opening in a direction opposite that of the needle slot of needle shaft, the coupler-housing slot configured to allow the access guidewire (106) to escape from the coupler housing when the introducer needle is withdrawn from the coupler, optionally
wherein the coupler-housing slot (226) has a narrower portion and a wider portion proximal of the narrower portion, the wider portion of the coupler-housing slot configured for at least aligning a needle-hub alignment rail (190) of the needle hub while assembling the RICC insertion assembly.

15. The RICC insertion assembly according to any of claims 11 to 14, wherein the access guidewire (106) includes an access-guidewire hub (250) over the distal end of the access guidewire, the access-guidewire hub configured to prevent over advancement of the access guidewire.

## Patentansprüche

1. Einführnadelbaugruppe (158), umfassend:
eine Einführnadel (104) einschließend:
einen Nadelschaft (162) einschließend einen longitudinalen Nadelschlitz (168), der sich von einem proximalen Abschnitt des Nadelschafts durch eine distale Nadelspitze (166) erstreckt;
eine Hülle (164) über dem Nadelschaft (162), die den Nadelschlitz (168) darunter abdichtet, ausgenommen den unter einer Hüllenöffnung (178) in einem proximalen Abschnitt der Hülle;
eine Nadelnabe (160) über den proximalen Abschnitten des Nadelschafts und der Hülle, wobei die Nadelnabe einen sich distal erstreckenden Nadelnabenverlängerungsarm (180) einschließt; und
einen Nadelnabenabschnitt (184) eines schnell einsetzbaren Zentralkatheter- ("RICC") -Clips (186), der ausgebildet ist, um einen Abschnitt eines RICC (102) darin zu halten; und
einen Koppler (108), der mit der Einführnadel gekoppelt ist, wobei der Koppler Folgendes einschließt:
ein Kopplergehäuse (212); und
einen Kopplerabschnitt (216) des RICC-Clips (186), der mit dem Kopplergehäuse verbunden ist.

2. Einführnadelbaugruppe nach Anspruch 1, wobei das Kopplergehäuse (212) eine sich proximal erstreckende Kopplergehäusestütze (214) einschließt, über die mindestens der Nadelnabenverlängerungsarm (180) verschiebbar angeordnet ist.

3. Einführnadelbaugruppe nach Anspruch 2, wobei der Kopplerabschnitt (216) des RICC-Clips (186) ein erster "C"-förmiger Abschnitt des RICC-Clips ist, der an einem proximalen Abschnitt der Kopplergehäusestütze (214) aufgehängt ist, der Nadelnabenabschnitt (184) des RICC-Clips (186) ein zweiter "C"-förmiger Abschnitt des RICC-Clips ist, der an einem proximalen Nadelnabenabschnitt (160) aufgehängt ist, und der erste und der zweite "C"-förmige Abschnitt des RICC-Clips in Richtung zueinander offen sind und dadurch den RICC-Clip vervollständigen.

4. Einführnadelbaugruppe nach einem der Ansprüche 1 - 3, wobei das Kopplergehäuse (212) eine Nadelnabenaufnahme (224) in einem proximalen Abschnitt des Kopplergehäuses einschließt, wobei in dieser Nadelnabenaufnahme ein distaler Abschnitt der Nadelnabe (160) einschließlich des Nadelnabenverlängerungsarms (180) angeordnet ist.

5. Einführnadelbaugruppe nach Anspruch 4, wobei der Koppler weiter ein Ventilmodul (218) einschließt, das in einem Ventilmodulfach (222) des Kopplergehäuses (212) distal zur Nadelnabenaufnahme (224) angeordnet ist, wobei das Ventilmodul um den proximalen Abschnitt der Hülle (164) und, wenn vorhanden, einen distalen Abschnitt eines Zugangsführungsdrahts (106), der sich durch die Hüllenöffnung erstreckt, abdichtet, wodurch eine leckagefreie Aspiration durch die Einführnadel ermöglicht wird.

6. Einführnadelbaugruppe nach Anspruch 5, wobei das Ventilmodul (218) eine integrierte Klinge (236) einschließt, die im Nadelschlitz (168) unterhalb eines distalen Endes der Hüllenöffnung (178) angeordnet ist, wobei die Klinge einen nach distal gerichteten Klingenrand einschließt, der ausgebildet ist, um die Hülle vom Nadelschaft abzuschneiden, wenn die Einführnadel aus dem Koppler zurückgezogen wird.

7. Einführnadelbaugruppe nach einem der Ansprüche 1 - 6, wobei der Koppler (108) ein Paar von Verriegelungsknöpfen (220) einschließt, die sich durch gegenüberliegende Seiten des Kopplergehäuses (212) erstrecken, wobei das Paar von Verriegelungsknöpfen jeweils auf einem Paar von Achsen (240) montiert ist, die im Kopplergehäuse (212) unverlierbar gehalten sind, sodass jeder Verriegelungsknopf des Paares von Verriegelungsknöpfen um eine entsprechende Achse schwenkt.

8. Einführnadelbaugruppe nach Anspruch 7, wobei jeder Verriegelungsknopf des Paares von Verriegelungsknöpfen (220) einen Vorsprung (242), der sich von einem Querarm (244) erstreckt, der den Vorsprung (242) mit einem äußeren, drückbaren Abschnitt des Verriegelungsknopfes verbindet, und einen Spalt gegenüber dem Querarm zwischen dem Vorsprung und dem äußeren, drückbaren Abschnitt des Verriegelungsknopfes aufweist.

9. Einführnadelbaugruppe nach Anspruch 8, wobei das Paar von Verriegelungsknöpfen (220) von einer Mittellinie des Kopplers (108) weg vorgespannt ist, sodass der Vorsprung (242) jedes Verriegelungsknopfes des Paares von Verriegelungsknöpfen (220) in einer Kerbe eines Paares von Kerben (194) in einer Wand des Nadelhüllenverlängerungsarms (180) auf gegenüberliegenden Seiten des Nadelhüllenverlängerungsarms in einem Grundzustand des Paares von Verriegelungsknöpfen angeordnet ist.

10. Einführnadelbaugruppe nach Anspruch 9, wobei die Wand des Nadelhüllenverlängerungsarms (180) auf beiden Seiten des Nadelhüllenverlängerungsarms den Spalt zwischen dem Vorsprung (242) und dem äußeren, drückbaren Abschnitt jedes Verriegelungsknopfes des Paares von Verriegelungsknöpfen (220) passiert, wenn das Paar von Verriegelungsknöpfen in Richtung der Mittellinie des Kopplers gedrückt wird.

11. Schnell einsetzbare Zentralkatheter- ("RICC") -Einsetzbaugruppe (100), umfassend:
einen RICC (102);
eine Einführnadelbaugruppe (158) nach einem der Ansprüche 1 bis 10, einen Zugangsführungsdraht (106) einschließend:
einen proximalen Abschnitt einschließend ein proximales Ende; und
einen distalen Abschnitt einschließend ein distales Ende, das in der Einführnadel (104) unmittelbar proximal zur Nadelspitze angeordnet ist, zumindest im einsatzbereiten Zustand der RICC-Einsetzbaugruppe; und
den Koppler (108), der den RICC (102), die Einführnadel (104), und den Zugangsführungsdraht (106) miteinander koppelt, wobei die proximalen und distalen Enden des Zugangsführungsdrahts (106) eine Schlaufe im Zugangsführungsdraht erzwingen, über die der RICC (102) mindestens im einsatzbereiten Zustand der RICC-Einsetzbaugruppe angeordnet ist.

12. RICC-Einsetzbaugruppe nach Anspruch 11, wobei die Nadelnabe (160) eine sich proximal erstreckende Nadelnabenwiege (182) mit Griffpolstern (196) auf gegenüberliegenden Seiten der Nadelnabenwiege (182) einschließt, um Halten der RICC-Einsetzbaugruppe (158) dadurch zu erleichtern, wenn die Einführnadel aus dem Koppler zurückgezogen wird, optional
weiter umfassend eine Spritze (110), die im mindestens einsatzbereiten Zustand der RICC-Einsetzbaugruppe (158) mit der Einführnadel (104) fluidisch gekoppelt ist, wobei ein Ventileinsatz (126) der Spritze (110) in der Nadelnabenwiege (182) mit Griffpolstern (196) angeordnet ist, die den Ventileinsatz der Spritze flankieren, weiter optional
ein weiterer RICC-Clip (112), der in eine Kolbenflanschverlängerung (114) integriert ist, die sich von einem Kolbenflansch (116) eines Kolbens (120) der Spritze erstreckt, wobei der weitere RICC-Clip ausgebildet ist, um eine Katheternabe (132) des RICC darin zu halten, sodass der RICC (102) mindestens im einsatzbereiten Zustand der RICC-Einsetzbaugruppe an der RICC-Einsetzbaugruppe aufgehängt ist.

13. RICC-Einsetzbaugruppe nach einem der Ansprüche 11 oder 12, wobei die Nadelnabe (160) einen Zugangsführungsdrahtkanal (188) einschließt, der durch eine Kerbe in einem sich distal erstreckenden Nadelnabenverlängerungsschlauch (189) über den proximalen Abschnitten des Nadelschafts und der Hülle definiert ist, optional
wobei die Nadelnabe einen Quersteg (198) und ein Nadelnabenpolster (202) zwischen dem Steg und dem Zugangsführungsdrahtkanal (188) einschließt, wobei der Steg und das Nadelnabenpolster ausgebildet sind zum Drücken und Halten des Zugangsführungsdrahts an Ort und Stelle dagegen, wenn ein Nadeltrakt von einem Bereich der Haut zu einem Blutgefäßlumen eines Patienten etabliert wird.

14. RICC-Einsetzbaugruppe nach einem der Ansprüche 11 bis 13, wobei das Kopplergehäuse (212) einen longitudinalen Kopplergehäuseschlitz (226) auf einer selben Seite des Kopplergehäuses wie der Kopplerabschnitt (216) des RICC-Clips (186) einschließt, wobei sich der Kopplergehäuseschlitz dadurch in eine Richtung entgegengesetzt der des Nadelschlitzes des Nadelschafts öffnet, und der Kopplergehäuseschlitz ausgebildet ist, um es dem Zugangsführungsdraht (106) zu erlauben, aus dem Kopplergehäuse zu entweichen, wenn die Einführnadel aus dem Koppler zurückgezogen wird, optional
wobei der Kopplergehäuseschlitz (226) einen schmaleren Abschnitt und einen breiteren Abschnitt proximal zum schmaleren Abschnitt aufweist, wobei der breitere Abschnitt des Kopplergehäuseschlitzes ausgebildet ist, um mindestens eine Nadelnabenausrichtungsschiene (190) der Nadelnabe während des Zusammenbaus der RICC-Einsetzbaugruppe auszurichten.

15. RICC-Einsetzbaugruppe nach einem der Ansprüche 11 bis 14, wobei der Zugangsführungsdraht (106) eine Zugangsführungsdrahtnabe (250) über dem distalen Ende des Zugangsführungsdrahts einschließt, wobei die Zugangsführungsdrahtnabe ausgebildet ist, um ein übermäßiges Vorschieben des Zugangsführungsdrahts zu verhindern.

## Revendications

1. Ensemble aiguille d'introduction (158), comprenant :
une aiguille d'introduction (104) incluant :
une tige (162) d'aiguille incluant une fente longitudinale (168) d'aiguille s'étendant depuis une partie proximale de la tige d'aiguille à travers une pointe distale (166) d'aiguille ;
une gaine (164) par-dessus la tige (162) d'aiguille scellant la fente (168) d'aiguille sous celle-ci, à l'exception d'une partie sous une ouverture (178) de gaine dans une partie proximale de la gaine ;
un raccord (160) d'aiguille par-dessus les parties proximales de la tige d'aiguille et de la gaine, le raccord d'aiguille incluant un bras d'extension (180) de raccord d'aiguille s'étendant de manière distale ; et
une partie raccord d'aiguille (184) d'un clip (186) de cathéter central à insertion rapide (« RICC ») configurée pour maintenir une partie d'un RICC (102) à l'intérieur de celle-ci ; et
un coupleur (108) couplé à l'aiguille d'introduction, le coupleur incluant :
un boîtier (212) de coupleur ; et
une partie coupleur (216) du clip (186) de RICC reliée au boîtier de coupleur.

2. Ensemble aiguille d'introduction selon la revendication 1, dans lequel le boîtier (212) de coupleur inclut un support (214) de boîtier de coupleur s'étendant de manière proximale par-dessus lequel au moins le bras d'extension (180) de raccord d'aiguille est disposé de manière coulissante.

3. Ensemble aiguille d'introduction selon la revendication 2, dans lequel la partie coupleur (216) du clip (186) de RICC est une première partie en forme de « C » du clip de RICC suspendue à une partie proximale du support (214) de boîtier de coupleur, la partie raccord d'aiguille (184) du clip (186) de RICC est une deuxième partie en forme de « C » du clip de RICC suspendue à une partie proximale du raccord (160) d'aiguille, et les première et deuxièmes parties en forme de « C » du clip de RICC s'ouvrent l'une vers l'autre, réalisant le clip de RICC.

4. Ensemble aiguille d'introduction selon l'une quelconque des revendications 1 à 3, dans lequel le boîtier (212) de coupleur inclut un réceptacle (224) de raccord d'aiguille dans une partie proximale du boîtier de coupleur, réceptacle de raccord d'aiguille dans lequel est disposée une partie distale du raccord (160) d'aiguille incluant le bras d'extension (180) de raccord d'aiguille.

5. Ensemble aiguille d'introduction selon la revendication 4, le coupleur incluant en outre un module de valve (218) disposé dans un compartiment (222) de module de valve du boîtier (212) de coupleur distal du réceptacle (224) de raccord d'aiguille, le module de valve assurant une étanchéité autour de la partie proximale de la gaine (164) et, lorsqu'elle est présente, d'une partie distale d'un fil-guide d'accès (106) qui s'étend à travers l'ouverture de gaine, permettant ainsi une aspiration sans fuite par l'aiguille d'introduction.

6. Ensemble aiguille d'introduction selon la revendication 5, dans lequel le module de valve (218) inclut une lame intégrée (236) disposée dans la fente (168) d'aiguille sous une extrémité distale de l'ouverture (178) de gaine, la lame incluant un bord de lame orienté distalement configuré pour couper la gaine de la tige d'aiguille lorsque l'aiguille d'introduction est retirée du coupleur.

7. Ensemble aiguille d'introduction selon l'une quelconque des revendications 1 à 6, dans lequel le coupleur (108) inclut une paire de boutons de verrouillage (220) s'étendant à travers des côtés opposés du boîtier (212) de coupleur, la paire de boutons de verrouillage étant respectivement montés sur une paire d'essieux (240) maintenus de manière captive dans le boîtier (212) de coupleur de telle sorte que chaque bouton de verrouillage de la paire de boutons de verrouillage pivote sur un essieu correspondant.

8. Ensemble aiguille d'introduction selon la revendication 7, dans lequel chaque bouton de verrouillage de la paire de boutons de verrouillage (220) présente une saillie (242) s'étendant depuis une traverse (244) reliant la saillie (242) à une partie externe pouvant être pressée du bouton de verrouillage et un espace en regard de la traverse entre la saillie et la partie externe pouvant être pressée du bouton de verrouillage.

9. Ensemble aiguille d'introduction selon la revendication 8, dans lequel la paire de boutons de verrouillage (220) est sollicitée à l'écart d'une ligne centrale du coupleur (108) de telle sorte que la saillie (242) de chaque bouton de verrouillage de la paire de boutons de verrouillage (220) est disposée dans une encoche d'une paire d'encoches (194) dans une paroi du bras d'extension (180) de raccord d'aiguille sur des côtés opposés du bras d'extension de raccord d'aiguille dans un état par défaut de la paire de boutons de verrouillage.

10. Ensemble aiguille d'introduction selon la revendication 9, dans lequel la paroi du bras d'extension (180) de raccord d'aiguille des deux côtés du bras d'extension de raccord d'aiguille traverse l'espace entre la saillie (242) et la partie externe pouvant être pressée de chaque bouton de verrouillage de la paire de boutons de verrouillage (220) lorsque la paire de boutons de verrouillage sont pressés vers l'intérieur en direction de la ligne médiane du coupleur.

11. Ensemble d'insertion (100) de cathéter central à insertion rapide (« RICC »), comprenant :
un RICC (102) ;
un ensemble aiguille d'introduction (158) selon l'une quelconque des revendications 1 à 10, un fil-guide d'accès (106) incluant :
une partie proximale incluant une extrémité proximale ; et
une partie distale incluant une extrémité distale disposée dans l'aiguille d'introduction (104) juste proximalement à la pointe d'aiguille dans au moins l'état prêt au déploiement de l'ensemble d'insertion de RICC ; et
le coupleur (108) couplant ensemble le RICC (102), l'aiguille d'introduction (104) et le fil-guide d'accès (106), les extrémités proximale et distale du fil-guide d'accès (106) imposant une boucle dans le fil-guide d'accès sur laquelle le RICC (102) est disposé dans au moins l'état prêt au déploiement de l'ensemble d'insertion de RICC.

12. Ensemble d'insertion de RICC selon la revendication 11, dans lequel le raccord (160) d'aiguille inclut un berceau (182) de raccord d'aiguille s'étendant de manière proximale pourvu de patins de préhension (196) sur des côtés opposés du berceau (182) de raccord d'aiguille pour faciliter le maintien de l'ensemble d'insertion (158) de RICC par celui-ci lorsque l'aiguille d'introduction est retirée du coupleur, facultativement
comprenant en outre une seringue (110) couplée de manière fluidique à l'aiguille d'introduction (104) dans au moins l'état prêt au déploiement de l'ensemble d'insertion (158) de RICC, un cylindre (126) de la seringue (110) étant disposé dans le berceau (182) de raccord d'aiguille avec les patins de préhension (196) flanquant le cylindre de la seringue, en outre facultativement
un autre clip (112) de RICC incorporé dans une extension (114) de bride de piston s'étendant depuis une bride (116) de piston d'un piston (120) de la seringue, l'autre clip de RICC étant configuré pour maintenir un raccord (132) de cathéter du RICC à l'intérieur de celui-ci de telle sorte que le RICC (102) est suspendu à l'ensemble d'insertion de RICC dans au moins l'état prêt au déploiement de l'ensemble d'insertion de RICC.

13. Ensemble d'insertion de RICC selon l'une quelconque des revendications 11 ou 12, dans lequel le raccord (160) d'aiguille inclut un canal (188) de fil-guide d'accès défini par une encoche dans un tube d'extension (189) de raccord d'aiguille s'étendant de manière distale sur les parties proximales de la tige d'aiguille et de la gaine, facultativement
dans lequel le raccord d'aiguille inclut une barre transversale (198) et un patin (202) de raccord d'aiguille entre la barre et le canal (188) de fil-guide d'accès, la barre et le patin de raccord d'aiguille étant configurés pour presser le fil-guide d'accès et le maintenir en place contre ceux-ci lors de l'établissement d'un trajet d'aiguille depuis une zone de peau jusqu'à une lumière de vaisseau sanguin d'un patient.

14. Ensemble d'insertion de RICC selon l'une quelconque des revendications 11 à 13, dans lequel le boîtier (212) de coupleur inclut une fente longitudinale (226) de boîtier de coupleur sur un même côté du boîtier de coupleur que la partie coupleur (216) du clip (186) de RICC, la fente de boîtier de coupleur s'ouvrant ainsi dans une direction opposée à celle de la fente d'aiguille de la tige d'aiguille, la fente de boîtier de coupleur étant configurée pour permettre au fil-guide d'accès (106) de s'échapper du boîtier de coupleur lorsque l'aiguille d'introduction est retirée du coupleur, facultativement
dans lequel la fente (226) de boîtier de coupleur présente une partie plus étroite et une partie plus large proximale de la partie plus étroite, la partie plus large de la fente de boîtier de coupleur étant configurée pour au moins aligner un rail d'alignement (190) de raccord d'aiguille du raccord d'aiguille lors de l'assemblage de l'ensemble d'insertion de RICC.

15. Ensemble d'insertion de RICC selon l'une quelconque des revendications 11 à 14, dans lequel le fil-guide d'accès (106) inclut un raccord (250) de fil-guide d'accès sur l'extrémité distale du fil-guide d'accès, le raccord de fil-guide d'accès étant configuré pour empêcher l'avancement excessif du fil-guide d'accès.
